# EUROPEAN PATENT APPLICATION

(11) **EP 4 591 942 A2**
(43) Date of publication of application: **30.07.2025**
(21) Application number: 25175722.5
(22) Date of filing: 07.07.2021
(51) Int. Cl.: A61P 37/08

(54) **INTERLEUKIN 33 (IL-33) INHIBITION FOR TREATMENT OF CANCER, FIBROSIS AND INFLAMMATION**

(30) Priority: 07.07.2020 US 202063048902 P
(62) Divisional of application: 21838227.3
(71) Applicant: The General Hospital Corporation, Boston, MA 02114 (US)
(72) Inventor: DEMEHRI, Shadmehr, Charlestown, 02129 (US); PARK, Jong Ho, Boston, 02108 (US)
(74) Representative: Forresters IP LLP

(57) **Abstract**

Described herein are methods and compositions for treating cancer, fibrosis, and inflammation, and reducing risk of developing cancer, fibrosis, and inflammation, using small molecule inhibitors of IL-33, i.e., pitavastatin calcium, tropisetron, ammo-nium glycyrrhizinate, ticagrelor, and cetrimonium bromide.

## Description

### CLAIM OF PRIORITY

This application claims the benefit of U.S. Provisional Application Serial No. 63/048,902, filed on July 7, 2020. The entire contents of the foregoing are incorporated herein by reference.

### FEDERALLY SPONSORED RESEARCH OR DEVELOPMENT

This invention was made with Government support under Grant No. AR068619 and AR076013 awarded by the National Institutes of Health. The Government has certain rights in the invention.

### TECHNICAL FIELD

Described herein are methods and compositions for treating cancer, fibrosis, and inflammation, and reducing risk of developing cancer, fibrosis, and inflammation, using small molecule inhibitors of IL-33, i.e., pitavastatin calcium, tropisetron, ammonium glycyrrhizinate, ticagrelor, and cetrimonium bromide.

### BACKGROUND

Chronic inflammation and its associated tumor-promoting environment are the hallmarks of many cancer types (Grivennikov *et al,* 2010; Todoric *et al,* 2016). Cancer-associated chronic inflammation contains immune cells and factors including regulatory T cells, myeloid-derived suppressor cells, interleukin (IL)-10, and transforming growth factor-beta (TGF-β) that exclude antitumor cytotoxic immunity (Grivennikov *et al.,* 2010; Todoric *et al.,* 2016). Thus, cancers that contain chronic inflammatory microenvironment are resistant to conventional chemotherapies and novel immunotherapies including immune checkpoint blockade resulting in poor prognosis (Brahmer *et al,* 2012; Johnson *et al,* 2017). To overcome this challenge, new approaches to prevent and treat cancer in the context of chronic inflammation are urgently needed.

### SUMMARY

The present invention is based, at least in part, on the discovery that IL-33 has a tumor-promoting role as a nuclear protein that is independent of its cytokine function. Nuclear IL-33 binds transcription factor RUNX2 thereby preventing RUNX2 from binding to *Smad6* promoter. By suppressing *Smad6* expression, nuclear IL-33 elevates p-SMAD2/3 and p-SMAD1/5 levels in the skin and pancreatic epithelial cells during chronic inflammation. The IL-33-dependent activation of the TGF-β/SMAD signaling pathway accelerates epithelial cell proliferation and contributes to cancer development in chronic inflammation. Therefore, blocking the nuclear IL-33/SMAD signaling axis with small molecule inhibitors is a novel strategy for cancer treatment and prevention, which can impact a large array of malignancies with increased IL-33 and SMAD signaling within the cancer cells and immune cells in the tumor microenvironment. In addition, small molecule IL-33 inhibitors is a novel strategy for the treatment of diseases like fibrosis and chronic inflammation (e.g., asthma, eczema and hepatitis) in which IL-33 and TGF-β/SMAD signaling are implicated.

Interleukin 33 (IL-33) in an epithelium-derived cytokine that is implicated in the pathogenesis of allergic inflammation like asthma and atopic dermatitis. As described herein, IL-33 is over-expressed in the context of chronic skin, colon and pancreas inflammatory diseases that are associated with high risk of cancer development. We have demonstrated that IL-33 deletion leads to prevention of cancer development in these organs. However, the deletion of its receptor, ST2 (Interleukin 1 Receptor Like 1 or IL1RL1), on immune cells leads to paradoxically more skin and pancreas tumors in chronic inflammation. Therefore, IL-33 inhibition (through the use of small molecule inhibitors, for example topically in the skin) is a therapeutic approach to prevent and treat cancers in the skin, colon, pancreas and perhaps other epithelial organs affected by high IL-33 expression in the epithelial cells proceeding and/or during cancer development.

Thus, provided herein are methods for treating inflammation associated with IL-33 activation in a subject. The methods comprise administering a therapeutically effective amount of an inhibitor selected from pitavastatin, tropisetron, ammonium glycyrrhizinate, ticagrelor, and cetrimonium bromide. Also provided are inhibitors selected from pitavastatin, tropisetron, ammonium glycyrrhizinate, ticagrelor, and cetrimonium bromide, for use in a method of treating inflammation associated with IL-33 activation in a subject.

Additionally, provided herein are methods of treating an allergic, fibrotic, infectious, or chronic inflammatory disease in a subject. The methods comprise administering a therapeutically effective amount of a pitavastatin, tropisetron, ammonium glycyrrhizinate, ticagrelor, and cetrimonium bromide. Also provided is an inhibitor selected from pitavastatin, tropisetron, ammonium glycyrrhizinate, ticagrelor, and cetrimonium bromide, for use in a method of treating an allergic, fibrotic, infectious, or chronic inflammatory disease in a subject.

In some embodiments, the chronic inflammatory condition is asthma, atopic dermatitis, allergic contact dermatitis, hepatitis, pancreatitis, or inflammatory bowel disease (IBD). In some embodiments, the subject is having anaphylaxis.

In some embodiments, the fibrotic disorder is organ fibrosis. In some embodiments, the organ fibrosis is fibrosis of skin (e.g., scleroderma, Dupuytren's disease (DD)), hepatic, pancreatic, renal, bowel (e.g., IBD-associated fibrosis), or pulmonary tissues.

Additionally provided herein are methods for treating or reducing risk of developing cancer in a subject. The methods comprise administering a therapeutically effective amount of a pitavastatin, tropisetron, ammonium glycyrrhizinate, ticagrelor, and cetrimonium bromide. In some embodiments, the subject has a fibrotic or chronic inflammatory disease (e.g., an inflammatory disease that increases risk of developing cancer above the level of the general public). In some embodiments, the chronic inflammatory condition is asthma, atopic dermatitis, allergic contact dermatitis, hepatitis, pancreatitis, or inflammatory bowel disease (IBD), or the fibrotic disorder is organ fibrosis. In some embodiments, the organ fibrosis is fibrosis of skin (e.g., scleroderma, Dupuytren's disease (DD)), hepatic, pancreatic, renal, bowel (e.g., IBD-associated fibrosis), or pulmonary tissues. Also provided herein is an inhibitor selected from pitavastatin, tropisetron, ammonium glycyrrhizinate, ticagrelor, and cetrimonium bromide, for use in a method of treating or reducing risk of developing cancer in a subject.

In some embodiments, the inhibitor is pitavastatin, and the subject does not have hyperlipidemia or mixed dyslipidemia.

In some embodiments, the inhibitor is tropisetron, and the subject does not have nausea and vomiting, or does not have fibromyalgia.

In some embodiments, the inhibitor is ticagrelor, and the subject does not have acute coronary syndrome or coronary artery disease (CAD).

Further provided herein are pharmaceutical compositions formulated for topical application comprising pitavastatin, tropisetron, ammonium glycyrrhizinate, and/or ticagrelor. In some embodiments, the pharmaceutical composition is an ointment, salve, gel, balm, lotion, milk, or cream. In some embodiments, the pharmaceutical compositions are for use in treating skin inflammation, skin fibrosis, or skin cancer in a subject.

Additionally provided herein are pharmaceutical compositions formulated for inhlataional administration application comprising pitavastatin, tropisetron, ammonium glycyrrhizinate, ticagrelor, and/or cetrimonium bromide. In some embodiments, the pharmaceutical composition is a mist, a fine powder, or an aerosol spray. In some embodiments, the pharmaceutical composition is packaged in a pressured container or dispenser, e.g., inhaler (e.g., metered dose inhaler (MDI)), insufflator, or nebulizer. In some embodiments, the pharmaceutical composition is for use in treating pulmonary inflammation (e.g., allergic asthma), pulmonary fibrosis, or lung cancer in a subject.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

### DESCRIPTION OF DRAWINGS

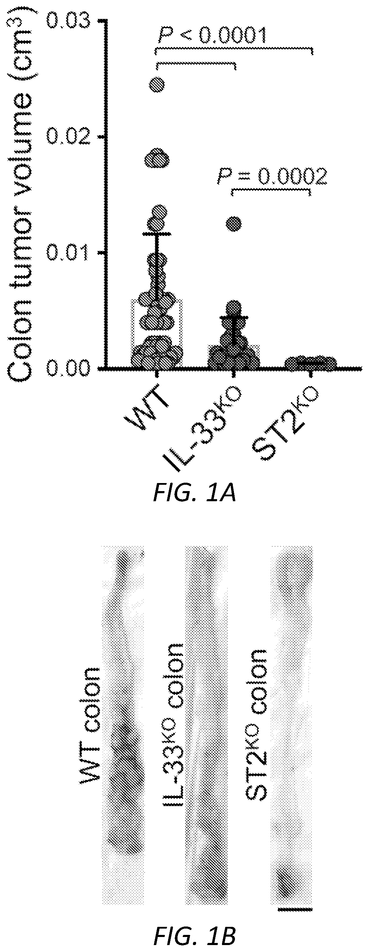

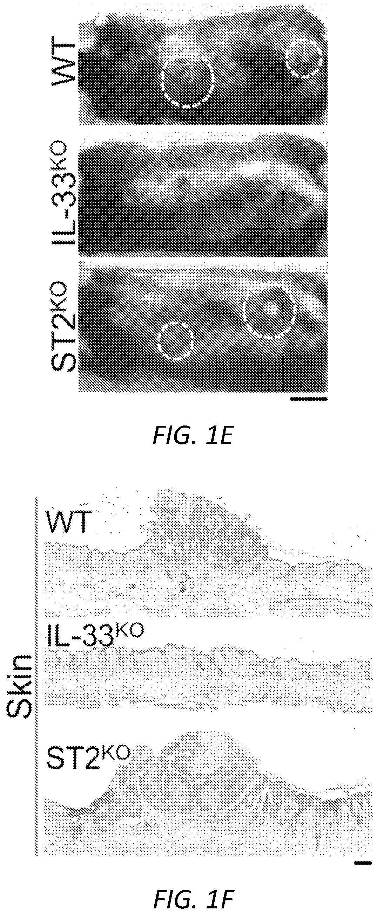

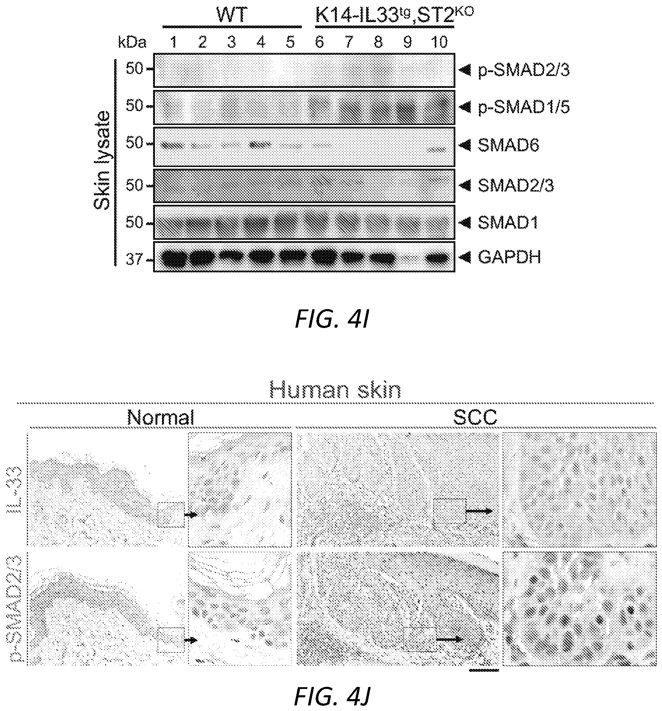

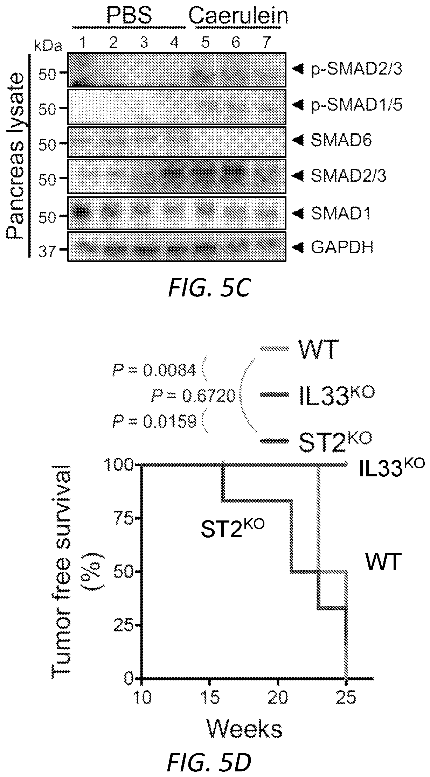

**FIGs. 1A-J****. IL-33 promotes skin cancer development in an ST2-independent manner.**
   **(A-C)** Colorectal tumor outcomes in WT (n=7), IL-33^{KO} (n=4) and ST2^{KO} (n=3) mice at the completion of the AOM/DSS carcinogenesis protocol. (A) Tumor volumes at the endpoint (each dot represents a tumor, mice with no tumor are marked as zero), (B) representative images of the exposed colonic lumen (scale bar: 1 cm), and (C) representative images of H&E-stained distal colon in each group (scale bar: 100 µm).
   **(D-F)** Skin tumor outcomes in WT (n=7), IL-33^{KO} (n=6) and ST2^{KO} (n=7) mice at the completion of the DMBA/DNFB carcinogenesis protocol. (D) Tumor volumes at the endpoint (each dot represents a tumor, mice with no tumor are marked as zero), (E) representative images of the back skin (yellow circles highlight the skin tumors, scale bar: 1 cm), and (F) representative images of H&E-stained back skin of the mice in each group (scale bar: 100 µm).
   **(G)** Representative images of IL-33 immunostaining on WT skin treated with DNFB or acetone control. Arrows in the inset highlight IL-33⁺ nuclei in epidermal keratinocytes (scale bar: 100 µm).
   **(H)** Quantification of nuclear IL-33⁺ epidermal and dermal cells in the DNFB-versus acetone-treated WT skin. Dots represent cell counts from three randomly selected high power field (HPF) images per sample (n=5 per group).
   **(I)** Representative images of IL-33 immunostaining on WT colon at the completion of the AOM/DSS carcinogen protocol compared with no treatment control. Arrows in the inset point to IL-33⁺ stromal cells and arrowheads point to lack of IL-33 nuclear stain in colonic epithelial cells (scale bar: 100 µm).
   **(J)** Quantification of nuclear IL-33⁺ epithelial and stromal cells of colon treated with AOM/DSS versus no treatment. Dots represent cell counts from three randomly selected HPF images per sample (n=4 per group).
   Data information: Graphs show mean + SD, unpaired t-test.

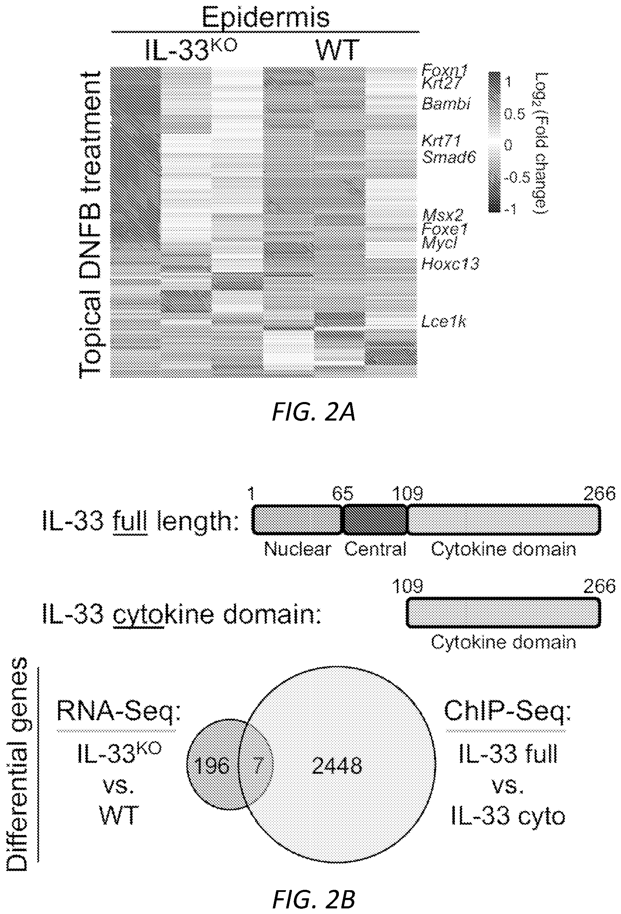

**FIGs. 2A-I**. **Nuclear IL-33 blocks *Smad6* expression via interaction with RUNX2 transcription factor.**
   (A) Heatmap of differentially expressed genes between IL-33^{KO} (n=3) and WT (n=3) epidermis after topical treatment with DNFB. Representative genes in differentially affected biological pathways are listed on the heatmap.
   (B) Schematic of IL-33 full length and IL-33 cytokine domain constructs (top). Venn diagram of differential genes shared between RNA-Seq and ChIP-Seq results (bottom). For ChIP-Seq, Pam212 cells transfected with IL-33 full length versus IL-33 cytokine domain were compared. Any gene with a peak in HA-empty vector control was excluded from the final analysis.
   **(C)** RNA-Seq result of the seven differential genes shared between RNA-Seq and ChIP-Seq results. Significance analysis comparing the two groups was performed using DESeq2 R package.
   ***(D)** Il33* and *Smad6* expression levels in DNFB-treated skin relative to acetone-treated controls (n=9 in DNFB group, n=7 in acetone group for *Il33,* n=10 in DNFB group, and n=9 in acetone group for *Smad6*)*.*
   ***(E)** Smad6* and *Pdcd4* (negative control gene) expression levels upon IL-33 full length versus cytokine domain expression compared with HA-empty vector expression in Pam212 cells (n=4 in each group).
   **(F)** Immunoblot for interaction between endogenous IL-33 and RUNX2. Lysate from PyMT and Pam212 cell lines were subjected to immunoprecipitation with an anti-IL-33 antibody followed by immunoblot analysis. Immunoprecipitation with an anti-IgG antibody on an equal amount of PyMT lysate is shown as a negative control. Data represent two independent experiments with similar results.
   **(G)** Immunoblot for interaction between nuclear IL-33 and RUNX2. After Pam212 cells were transfected with IL-33 full length or cytokine domain for 24 hours, cell lysates were subjected to immunoprecipitation with anti-IL-33 antibody followed by immunoblot analysis.
   **(H)** ChIP-qPCR assay for *Smad6* in the presence of IL-33 full length or cytokine domain using an anti-RUNX2 antibody. After Pam212 cells were transfected with IL-33 full length or cytokine domain for 24 hours, cell lysates were subjected to chromatin-immunoprecipitation with anti-RUNX2 antibody and eluted RUNX2-bound chromatin was used for qPCR with primers against *Smad6* promoter region. Anti-IgG ChIP-qPCR results are shown as negative controls (n=4 per group).
   **(I)** The experimental design used to induce chronic skin inflammation with repeated topical DNFB treatment. At the completion of the 22-day treatment period, epidermis samples were collected for RNA-Seq.
   Data information: GAPDH is used as the control housekeeping protein in (F) and (G). Graphs show mean + SD, NS: not significant, unpaired *t*-test.

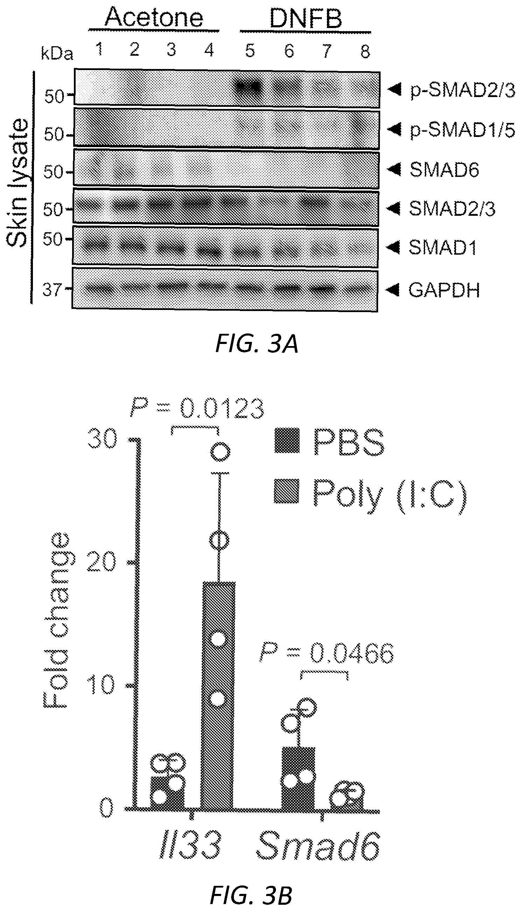

**FIGs. 3A-J**. **Nuclear IL-33 promotes cell proliferation by upregulating TGF-β/SMAD signaling pathway.**
   **(A)** Immunoblot of p-SMAD2/3, p-SMAD1/5, SMAD6, SMAD2/3 and SMAD1 proteins in DNFB- versus acetone-treated WT skin (n=4 in each group).
   **(B)** Quantitative PCR (qPCR) of *Il33* and *Smad6* mRNA in Pam212 cells treated with poly (I:C) or PBS (carrier control, n=4 in each group).
   **(C)** ChIP-qPCR assay for *Smad6* in the presence of poly (I:C) or PBS using an anti-IL-33 antibody. After Pam212 cells were transfected with poly (I:C) for 24 hours, cell lysates were subjected to chromatin-immunoprecipitation with anti-IL-33 antibody and eluted IL-33-bound chromatin was used for qPCR with *Smad6* regulatory peak region (63,968k) primers and *Smad6* no peak region (63,955k) primers (n=4 in each group).
   **(D)** Immunoblot of p-SMAD2/3 and SMAD2/3 proteins in response to TGF-β and poly (I:C) treatment. Pam212 cells were treated with poly (I:C) or PBS followed by incubation with and without TGF-β (5nM). Data represent three independent experiments with similar results.
   **(E)** Immunoblot of p-SMAD2/3 and SMAD2/3 proteins in response to TGF-β + poly (I:C) after knocking down of IL-33. Pam212 cells were transfected with siIl33 knockdown construct or siRNA control (siCon) for 30 hours followed by incubation with poly (I:C) and TGF-β. Data represent three independent experiments with similar results.
   **(F)** Immunoblot of p-SMAD2/3 and SMAD2/3 proteins upon the expression of IL-33 full length or cytokine domain. Pam212 cells were transfected with IL-33 full length or cytokine domain for 24 hours followed by incubation with and without TGF-β. Data represent three independent experiments with similar results.
   **(G)** Impact of IL-33 knockdown on cell proliferation in response to TGF-β. Pam212 cells were treated with siIl33 or siCon followed by TGF-β treatment (n=7 in each group).
   **(H)** Impact of IL-33 knockdown and SB431542 treatment on cell proliferation. Pam212 cells were treated with siIl33 or siCon in combination with SB431542 or DMSO (carrier control, n=7 in each group).
   **(I)** Representative images of H&E- and Ki67-stained back skin of IL-33^{KO} and WT mice treated with 22-day DNFB protocol in combination with SB431542 or acetone (carrier control). Note the epidermal thickness, keratinocytes proliferation and dermal inflammation in each group (scale bars: 100 µm).
   **(J)** The epidermal thickness of DNFB-treated IL-33^{KO} and WT skin after treatment with SB431542 or acetone. Each dot represents the average of three measurements in an HPF image. 10 random HPF images per skin sample are included (n=10 for WT with acetone, n=8 for WT with SB431542, n=6 for IL-33^{KO} with acetone, n=5 for IL-33^{KO} with SB431542 per group).
      Data information: GAPDH is used as the control housekeeping protein in (A, D-F). Graphs show mean + SD, NS: not significant, unpaired t-test.

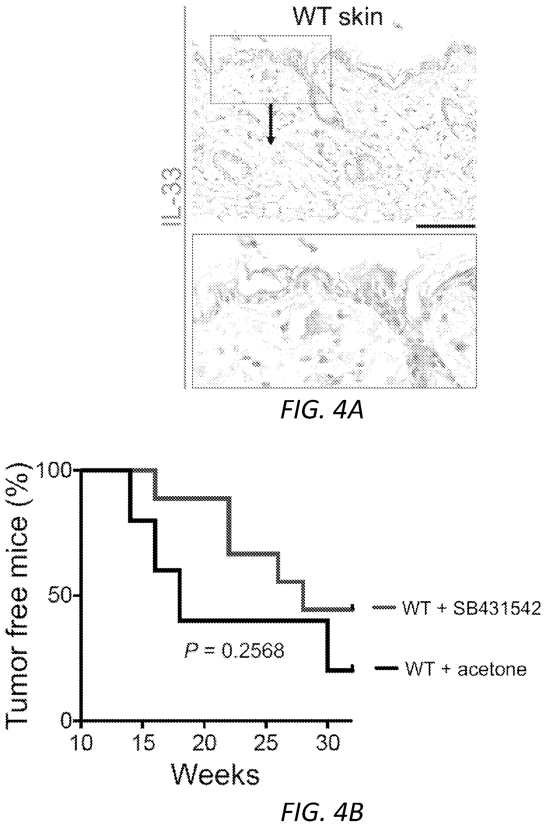

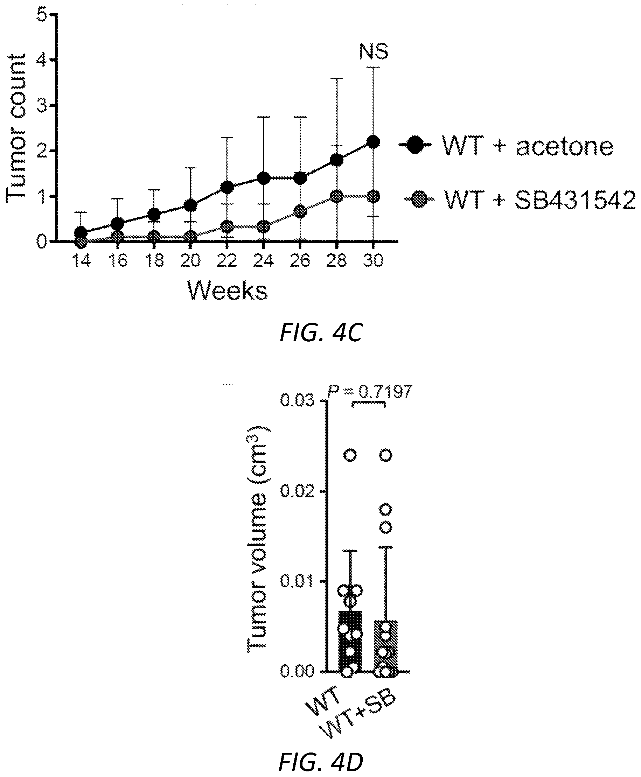

**FIGs. 4A-J** **Nuclear IL-33/SMAD signaling promotes skin cancer development.**.
   **(A)**Representative images of IL-33 immunostaining on WT skin. Inset highlights the IL-33-negative nuclei in the epidermis (scale bar: 100 µm).
   **(B-D)** Skin tumor outcomes in WT mice treated with DMBA/TPA carcinogenesis protocol in combination with SB431542 (test, n=9) or acetone (carrier control, n=5). (B) Tumor latency (log-rank test), (C) skin tumor counts per mouse over time, and (D) skin tumor volume at the completion of the DMBA/TPA carcinogenesis protocol.
   **(E)**Representative images of IL-33 immunostaining on K14-IL33^{tg},ST2^{KO} skin. Inset highlights the IL-33-positive nuclei in the epidermis (scale bar: 100 µm).
   **(F-H)** Skin tumor outcomes in K14-IL33^{tg},ST2^{KO} mice treated with DMBA/TPA carcinogenesis protocol in combination with SB431542 (test, n=6) or acetone (control, n=5). (F) Tumor latency (log-rank test), (G) skin tumor counts per mouse over time, and (H) skin tumor volume at the completion of the DMBA/TPA carcinogenesis protocol.
   **(I)**SMAD signaling proteins levels in WT and K14-IL33^{tg},ST2^{KO} skin treated with acetone (control) at the completion of the DMBA/TPA carcinogenesis protocol. Tissue lysates prepared from the whole back skin of the animals were subjected to immunoblot with p-SMAD2/3, p-SMAD1/5, SMAD6, SMAD2/3 and SMAD1 antibodies. GAPDH is used as the control housekeeping protein (n=5 in each group).
   **(J)**Representative images of IL-33 and p-SMAD2/3 immunostaining on the adjacent sections of human squamous cell carcinoma (SCC) and normal skin (scale bar: 100 µm).
   Data information: Graphs show mean + SD, NS: not significant, unpaired *t*-test.

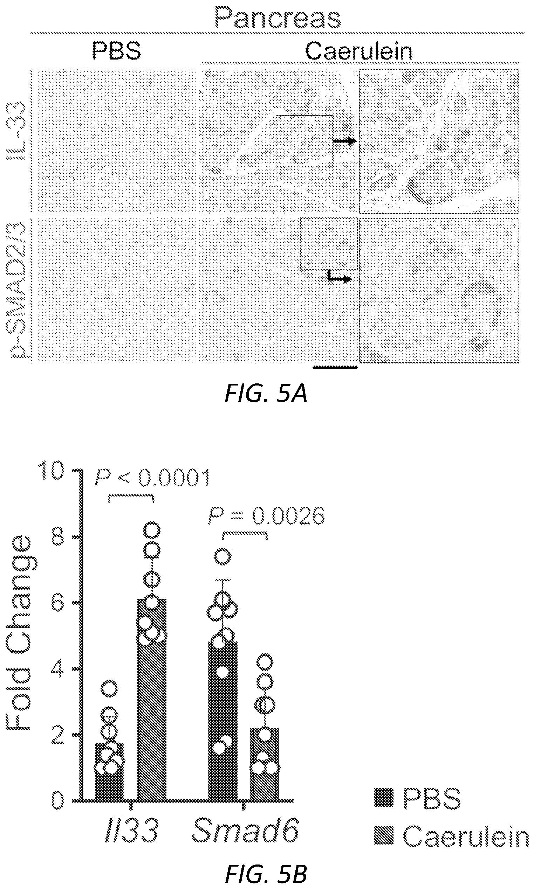

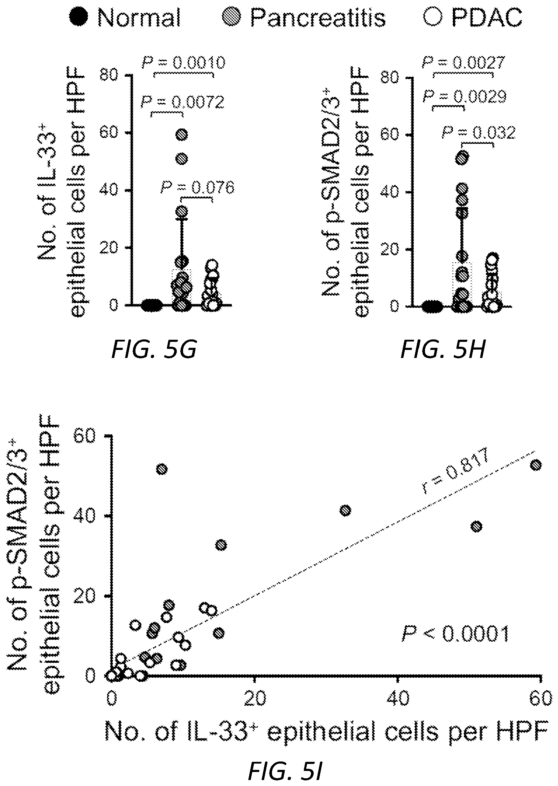

**FIGs. 5A-I**. **Nuclear IL-33 promotes pancreatic cancer development.**
   **(A)** Representative images of IL-33 and p-SMAD2/3 immunostaining on WT pancreas treated with caerulein or PBS (carrier control) to induce chronic pancreatitis (scale bar: 100 µm).
   ***(B)** Il33* and *Smad6* mRNA levels in caerulein-treated WT pancreas compared with PBS-treated controls at the completion of chronic pancreatitis protocol (n=8 in caerulein and n=9 in PBS group for *Il33,* n=9 in caerulein and n=10 in PBS group for *Smad6,* unpaired *t*-test).
   **(C)** Immunoblot for p-SMAD2/3, p-SMAD1/5, SMAD6, SMAD2/3 and SMAD1 proteins in lysates from caerulein (n=3) and PBS (n=4) treated WT pancreas. GAPDH is used as the control housekeeping protein.
   **(D)** Pancreatic tumor-free survival of WT (n=4), IL-33^{KO} (n=5) and ST2^{KO} (n=6) mice at the completion of the DMBA/caerulein carcinogenesis protocol (log-rank test).
   **(E)** Representative H&E images of two pancreatic tissues from WT, IL-33^{KO} and ST2^{KO} groups treated with DMBA/caerulein (scale bar: 100 µm).
   **(F)** Representative images of IL-33 and p-SMAD2/3 immunostaining on adjacent sections of matched normal pancreas, chronic pancreatitis and PDAC collected from pancreatic cancer patients (scale bar: 100 µm).
   **(G, H)** Quantification of nuclear (G) IL-33⁺ and (H) p-SMAD2/3⁺ epithelial cells per HPF in the matched human pancreatic tissues. Each dot represents the average cell counts across three randomly selected HPF images per sample (n=18 patients, paired t-test).
   **(I)** Correlation between IL-33⁺ and p-SMAD2/3⁺ epithelial cell counts across normal pancreas, chronic pancreatitis and PDAC samples (n=54 matched samples from 18 patients, *t*-test for the Pearson correlation coefficient).
   Data information: Graphs show mean + SD.

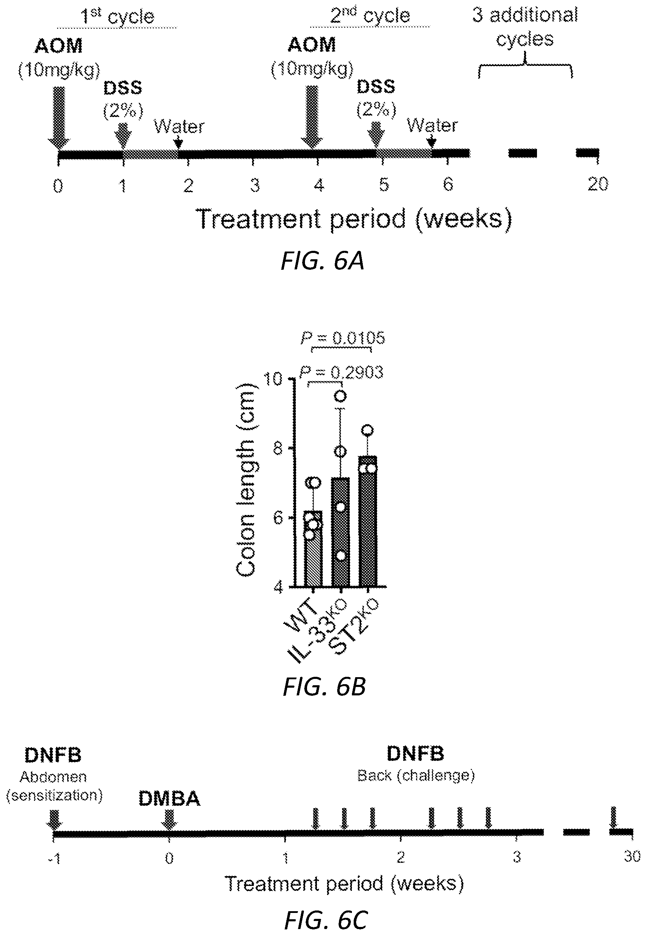

**FIGs. 6A-F**. **IL-33/ST2 impact on colorectal and skin carcinogenesis in chronic inflammation.**
   **(A)**Schematic diagram of colitis-induced colorectal carcinogenesis protocol in mice, consisting of five AOM/DSS treatment cycles over 20 weeks.
   **(B)**Colon length measurement, from cecum to rectum, in WT (n=6), IL-33^{KO} (n=4) and ST2^{KO} (n=3) mice at the completion of the AOM/DSS carcinogenesis protocol.
   **(C)**Experimental design for chronic inflammation-induced skin carcinogenesis consisting of an initial sensitization to DNFB allergen on the abdomen followed by treatment with a single dose of DMBA followed by DNFB challenges on the back skin three times a week for 29 weeks.
   **(D** and **E)** Skin tumor outcome in WT (n=7), ST2^{KO} (n=7) and IL-33^{KO} (n=6) mice treated with DMBA/DNFB carcinogenesis protocol. (D) Time to tumor onset (log-rank test) and (E) the number of skin tumors over time.
   **(F)**Representative images of IL-33 immunostained DNFB-treated skin and AOM/DSS-treated colon tissues from IL-33^{KO} mice (scale bars: 100 µm).
   Data information: Graphs show mean + SD, NS: not significant, unpaired t-test.

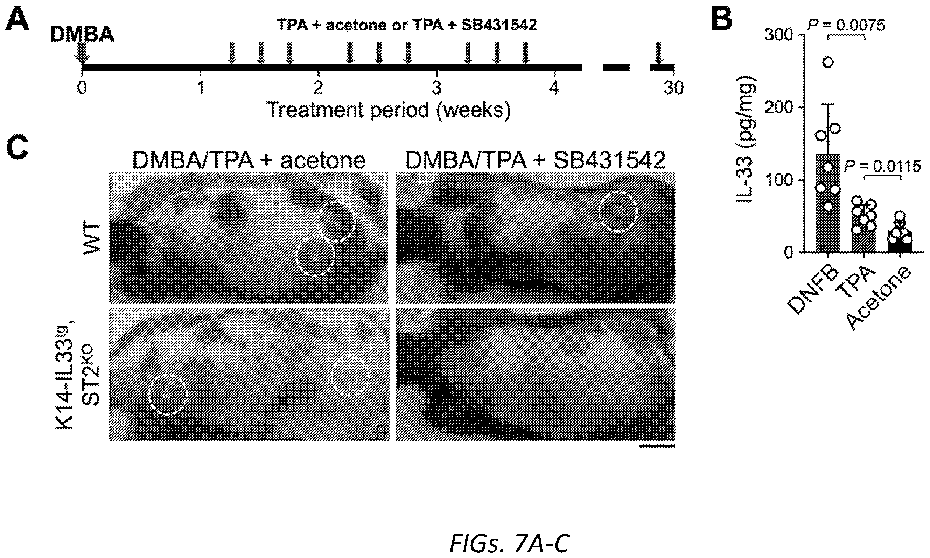

**FIGs. 7A-E**. **Blocking TGF-β/SMAD signaling attenuates nuclear IL-33 induced skin cancer development.**
   **(A)** Experimental design for DMBA/TPA skin carcinogenesis protocol in combination with TGF-β/SMAD inhibitor, SB431542, treatment.
   **(B)** IL-33 protein levels in TPA- and DNFB-treated skin compared with acetone (carrier)-treated controls (n=7 in each group, graph shows mean + SD, unpaired t-test).
   **(C)** Representative images of WT and K14-IL33^{tg},ST2^{KO} mice treated with SB431542 or acetone (carrier control) at the completion of DMBA/TPA carcinogenesis protocol (yellow circles highlight the skin tumors).
   **(D)** Representative images of IL-33 and p-SMAD2/3 immunostained adjacent skin sections from SB431542 and acetone-treated K14-IL33^{tg},ST2^{KO} mice that completed DMBA/TPA carcinogenesis protocol. Arrows point to IL-33⁺ and p-SMAD2/3⁺ epidermal keratinocytes. Arrowheads highlight keratinocytes that lack both IL-33 and p-SMAD2/3.
   **(E)** Representative images of Ki67 and cleaved caspase-3 immunofluorescence staining on the adjacent sections of SB431542 and acetone-treated K14-IL33^{tg},ST2^{KO} skin that underwent DMBA/TPA carcinogenesis protocol.
   Data information: Scale bars: mouse 1cm, tissue 100 µm.

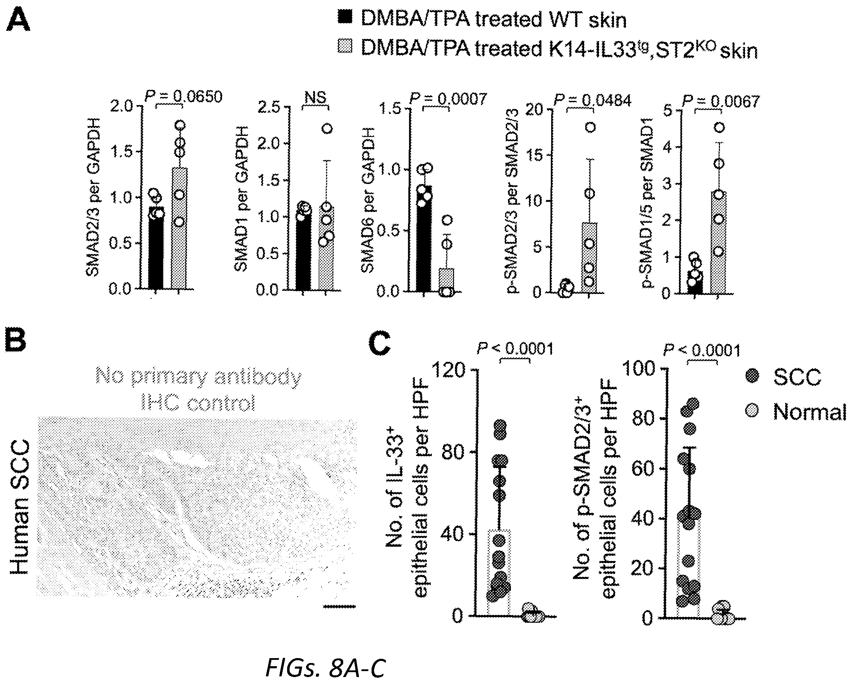

**FIGs. 8A-C**. **IL-33 and p-SMAD2/3 are highly expressed in human SCC.**
   **(A)** Quantification of protein bands shown in Figure 4I.
   **(B)** Representative image of negative control (no primary antibody) immunostaining on the adjacent tissue section of the human SCC sample shown in Figure 4J (scale bar: 100 µm).
   **(C)** Quantification of nuclear IL-33⁺ and p-SMAD2/3⁺ epithelial cells per HPF in SCC versus normal human skin. Dots represent cell counts in three randomly selected HPF images per sample across 5 SCC and 5 normal skin samples.
   Data information: Graphs show mean + SD, NS: not significant, unpaired t-test.

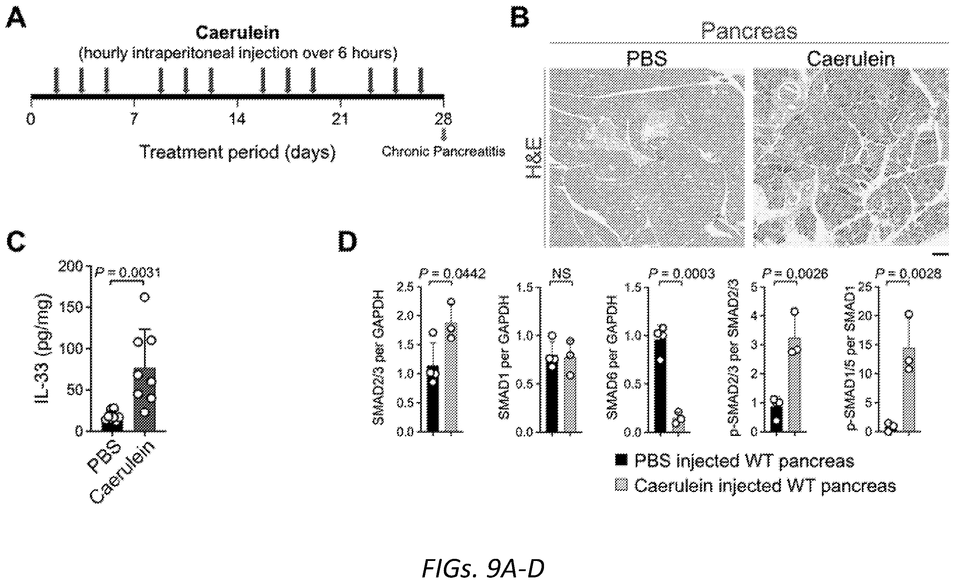

**FIGs 9A-F**. **Nuclear IL-33 promotes pancreatic cancer development in chronic pancreatitis.**
   **(A)** Experimental design for the induction of chronic pancreatitis in mice.
   **(B)** Representative images of H&E-stained pancreas of WT mice treated with caerulein versus PBS (carrier control) for 4 weeks (scale bar: 100 µm).
   **(C)** IL-33 protein levels in caerulein- compared with PBS-treated pancreas.
   **(D)** Quantification of protein bands shown in Figure 5C.
   **(E)** Experimental design for DMBA/caerulein-induced pancreatic cancer including an intrapancreatic injection with DMBA followed by intraperitoneal caerulein injections once per day, three times a week for 24 weeks.
   **(F)** Representative images of DMBA/caerulein-treated WT, IL-33^{KO} and ST2^{KO} mice and H&E-stained pancreas of WT, IL-33^{KO} and ST2^{KO}. Red arrows point to pancreatic tumor mass under the skin of WT and ST2^{KO} mice. Note the replacement of pancreas with large fibrotic tumors in WT and ST2^{KO} mice (scale bar: 1 cm).
   Data information: Graphs show mean + SD, NS: not significant, unpaired t-test.

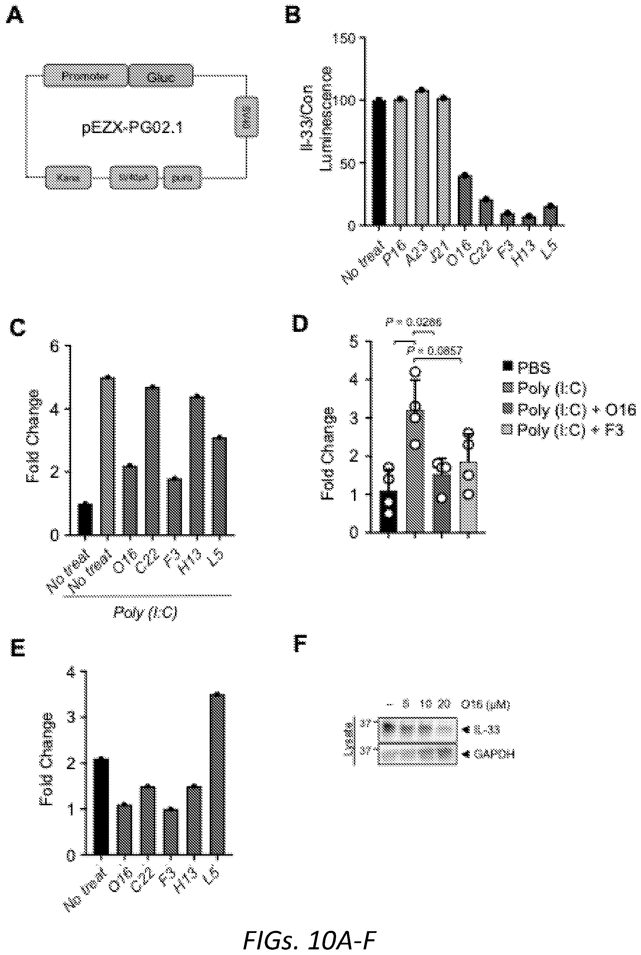

**FIGs. 10A-F**. **IL-33 small molecule inhibitor discovery.**
   (A) Exemplary construct with mouse Il33 gene promoter for small molecule library screening.
   (B) The candidate compounds the inhibitory effect against IL-33 reporter.
   (C) The effect of 5 candidate compounds on Il33 expression induced by Ploy (I:C) in Pam212 cells.
   (D) Confirmation of 016 (pitavastatin) and F3 (tropisetron) ability to block II33 expression in Poly-IC treated Pam212.
   (E) The inhibitory effect of 5 candidates on endogenous Il33 expression in PyMT breast cancer cell line.
   (F) IL-33 protein levels in PyMT cell after treatment with pitavastatin in a dose range.

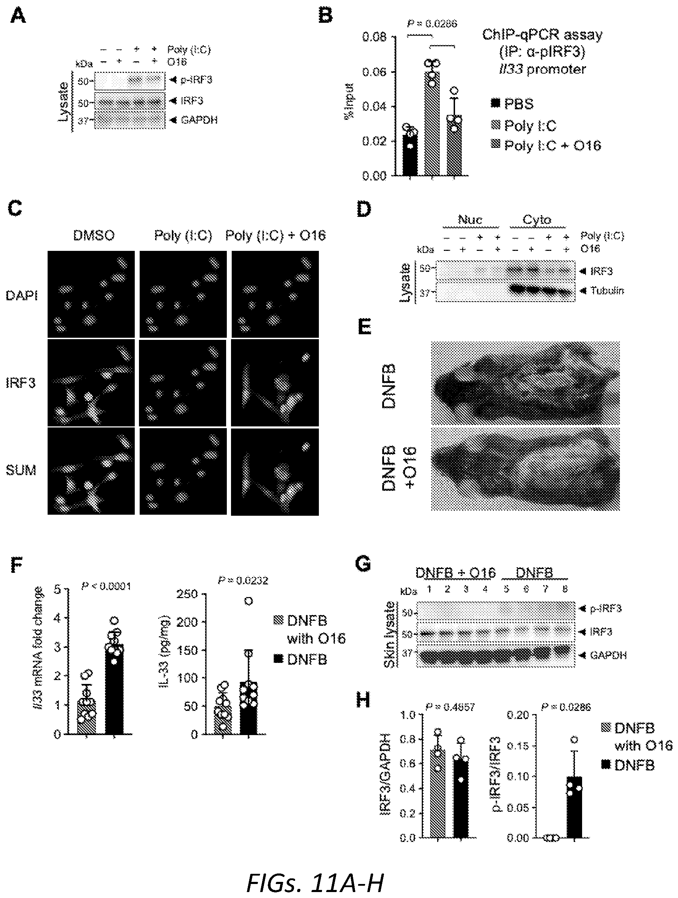

**FIGs. 11A-H**. **Mechanism and effect of IL-33 inhibitor.**
   **(A)** The effect of pitavastatin (016) to poly (I:C) induced p-IRF3 expression in Pam212 cells.
   **(B)** The effect of pitavastatin to p-IRF3 bindings to *Il33* promoter region in Pam212 cells.
   **(C)** The effect of pitavastatin to IRF3 nuclear localization in poly (I:C) treated Pam212 cells.
   **(D)** Blocking effect of pitavastatin with IRF3 localization in poly (I:C) mediated IRF3 nuclear translocation.
   **(E)** Representative images of DNFB treated mice back skin with or without pitavastatin treatment.
   **(F)** IL-33 protein and RNA levels in DNFB treated and DNFB with pitavastatin treated mice group.
   **(G)** The effect of pitavastatin to IRF3 pathway *in vivo* model.
   **(H)** Quantification of IRF3 and p-IRF3 protein level compared to GAPDH and IRF3 respectively from (G).

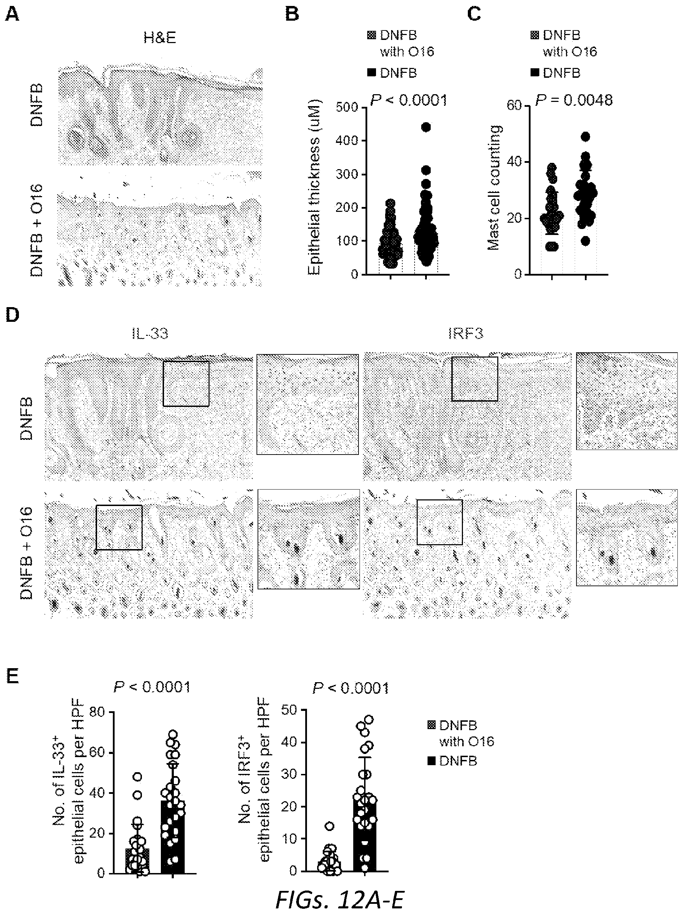

**FIGs. 12A-E**. **Effect of pitavastatin *in vivo* model.**
   **(A)** Representative images of H&E staining of DNFB-treated mouse back skin with or without pitavastatin treatment.
   **(B)** Quantification of epidermal thickness.
   **(C)** Quantification of dermal mast cells in the test versus control group.
   **(D)** Representative images of IL-33 and IRF3 staining of DNFB-treated mouse back skin with or without pitavastatin treatment.
   **(E)** Quantification of nuclear IL-33 and IRF3 staining epithelial cells per HPF in the skin tissues.
**FIG. 13****. Suppression of chronic pancreatitis by pitavastatin *in vivo.***
   Representative images of H&E staining of Caerulein-injected mouse pancreas with or without pitavastatin treatment. Note the reduction of inflammatory cell infiltrates (dark small cells around pancreatic epithelial cells) in the pitavastatin-treated pancreas.

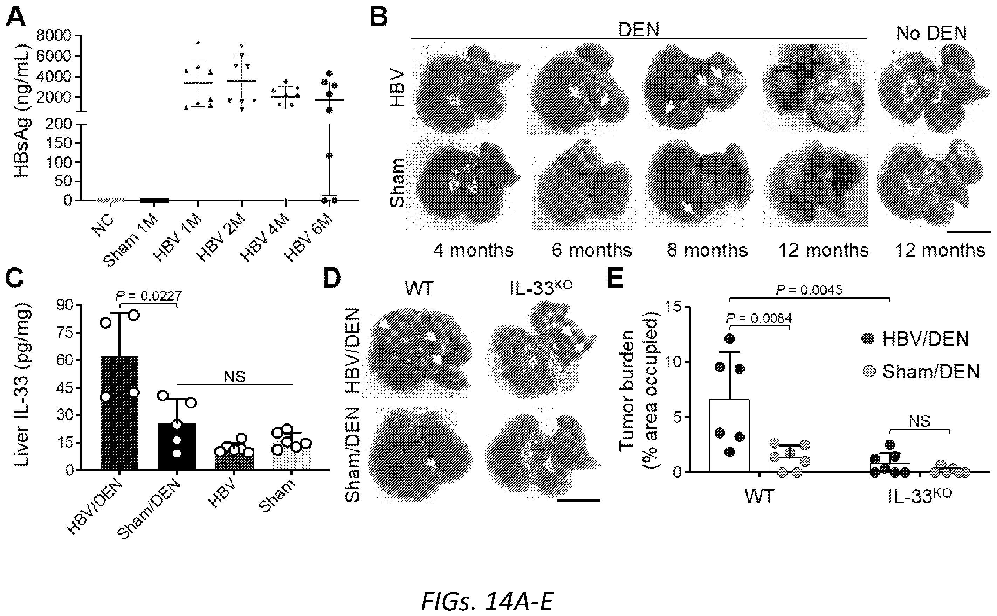

**FIGs. 14A-E**. **Chronic HBV infection combined with DEN treatment upregulates IL-33 expression and promotes cancer development in the liver.**
   **(A)** HBsAg detection in the serum of pAAV-HBV1.2-infected mice compared with empty vector (Sham) and no infection controls (NC) over time.
   **(B)** Macroscopic images of liver at 4, 6, 8 and 12 months pos-infection. Note that mice that did not receive DEN have no liver lesion at 12 months post-infection.
   **(C)** IL-33 protein levels in the hepatocytes isolated from the liver of HBV/DEN compared to sham/DEN, HBV and sham groups.
   **(D)** Macroscopic images of WT and IL-33^{KO} liver treated with HBV/DEN versus sham/DEN at 8 months post-infection.
   **(E)** HCC burden in the liver as measured by % liver surface area occupied by the tumors in WT and IL-33^{KO} liver treated with HBV/DEN versus sham/DEN at 8 months post-infection. Yellow arrows point to HCC foci on the liver, NS: not significant, student's *t* test, scale bars: 1 cm.

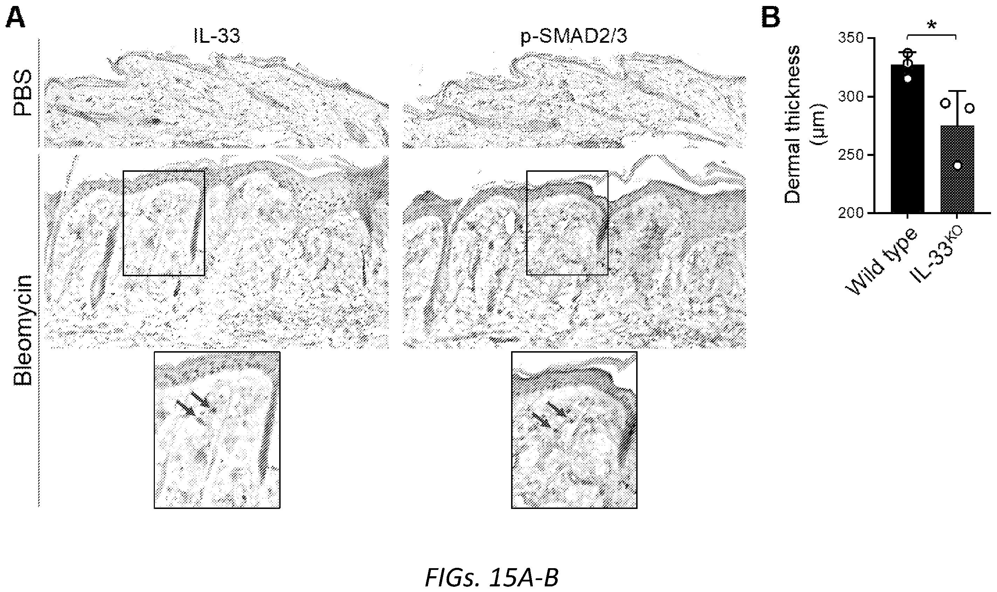

**FIGs. 15A-B**. **IL-33 overexpression in dermal fibroblasts drives skin fibrosis.**
   (A) IL-33 and phospho-SMAD2/3 expression in dermal fibroblasts of a wild-type skin treated with bleomycin compared with PBS (carrier) control. Note the co-localization of IL-33 and p-SMAD2/3 in adjacent tissue sections (arrows in insets).
   (B) Dermal thickness in bleomycin-treated wild-type versus IL-33 knockout (IL-33KO) mice. * p < 0.05, unpaired t test.

### DETAILED DESCRIPTION

TGF-β, bone morphogenetic protein (BMP), and SMAD signaling pathway are involved in many cellular processes, including tumorigenesis in chronic inflammation (Grivennikov *et al.,* 2010; Massague, 2012; Massague *et al,* 2000; Shi & Massague, 2003). TGF-β/BMP ligands bind to their receptors on the cell surface and activate SMAD2/3 or SMAD1/5 proteins through phosphorylation, followed by their translocation into the nucleolus by Co-SMAD (SMAD4) (Massague *et al,* 2005). SMAD6/7 are inhibitory SMADs (I-SMAD) that either block SMAD4 binding to other SMAD proteins or inactivate TGF-β/BMP receptor serine/threonine kinases (Imamura *et al,* 1997; Massague *et al.,* 2005). SMAD6 can block TGF-β/BMP-mediated tumorigenesis and is associated with an increased survival rate among patients with squamous cell carcinoma (SCC) (Mangone *et al,* 2010). TGF-β/BMP signaling is highly upregulated in pancreatic ductal adenocarcinoma (PDAC) (Bardeesy *et al,* 2006), which commonly arises in the context of chronic pancreatitis and is characterized by rapid progression and profound resistance to immunotherapy (Melzer *et al,* 2017; Shen *et al,* 2017). Thus, blocking TGF-β/BMP/SMAD signaling may provide an effective strategy to suppress carcinogenesis in chronic inflammation.

IL-33 is an epithelium-derived cytokine that is upregulated early on in cancer-prone chronic inflammation (Ameri *et al,* 2019). As a member of the IL-1 family of cytokines, IL-33 is implicated in the pathogenesis of allergic diseases by inducing a type 2 immune response (Saenz *et al,* 2008; Sy & Siracusa, 2016; Wang *et al,* 2018). In addition, IL-33 has been associated with pro and antitumor functions in cancer (Ameri *et al.,* 2019; Moral *et al,* 2020). Upon cellular stress, injury or necrosis, IL-33 is rapidly released to "alarm" the immune system (Molofsky *et al,* 2015). Secreted IL-33 binds to its cognate receptor, interleukin-1 receptor-like 1 (IL1RL1) or suppressor of tumorigenesis 2 (ST2 (also known as IL-1R4)), generating a potent pro-inflammatory stimulus particularly in the context of chronic inflammation (Kumar *et al,* 1995; Moussion *et al,* 2008; Oshikawa *et al,* 2002). Notably, IL-33 protein also has a nuclear localization signal and a homeodomain (helix-turn-helix-like motif) that can bind to heterochromatin in the nucleus, similar to nuclear proteins (Ali *et al,* 2011; Carriere *et al,* 2007). At baseline, IL-33 resides in the nucleus of the cells in which it is expressed. Therefore, IL-33 may have a dual function in epithelial cancers, acting as a cytokine when released from the cell and as a nuclear factor within the cell with transcriptional regulatory properties.

Previous studies have shown that IL-33 can act as a transcriptional repressor by inhibiting NF-κB protein and thereby dampening its transcription activity *in vitro* and function as histone methyltransferase in endothelial cells (Ali *et al.,* 2011; Carriere *et al.,* 2007). In addition, the cell-intrinsic role of IL-33 in regulatory T cells is found to be essential for their suppressive function in the tumor microenvironment (Hatzioannou *et al,* 2020). However, IL-33 is mostly known as an epithelium-derived alarmin cytokine in allergic diseases and cancer (Chan *et al,* 2019; He *et al,* 2017). As demonstrated herein, the N-terminal domain of IL-33, which interacts with RUNX2, plays an essential role in nuclear IL-33's regulation of the SMAD signaling pathway. Thus, the present discovery of IL-33 nuclear function in epithelial cells of skin and pancreas provides novel insights into the pathogenesis of cancer and inflammation in these organs. Ultimately, this work reveals that nuclear IL-33 functions to potentiate TGF-β/SMAD signaling in epithelial cells by repressing *Smad6* expression via interaction with RUNX2. The novel cell-autonomous effect of IL-33 in cancer cells suggests an innovative therapeutic path to blocking cancer development in chronic inflammation by targeting the IL-33/SMAD signaling axis with small molecules instead of antibodies against IL-33 cytokine.

The present work provides a much-needed insight into the pathogenesis of pancreatic cancer. It is well-established that pancreatic cancer arises in the context of chronic inflammation and contains a severely immunosuppressive environment nonresponsive to immunotherapeutics (Torphy *et al,* 2018). TGF-β/SMAD signaling is found to be upregulated in PDAC (Perera & Bardeesy, 2015). This signaling pathway can induce cell proliferation, angiogenesis and epithelial-mesenchymal transition (Colak & Ten Dijke, 2017; Jakowlew, 2006). In addition, the inhibition of SMAD signaling reduces PD-L1 and PD-L2 expression, which indicates the importance of this signaling pathway in creating an immunosuppressive microenvironment (Martinez *et al,* 2014). However, it is still unclear how this pathway is regulated in pancreatic cancer. The present results provide a mechanism for this upregulation by demonstrating that nuclear IL-33, which is induced in the inflamed epithelia, promotes TGF-β/SMAD signaling within the cancer cells. The majority of IL-33 and p-SMAD2/3 proteins are localized in the nuclei of the pancreatic acinar cells in cancer-prone chronic pancreatitis of mice and humans. Pancreatic acinar cells are considered the prime origin of PDAC (Guerra *et al.,* 2007). Therefore, the present findings suggest that the IL-33/SMAD signaling axis is involved in the transformation of acinar cells into cancer cells that form in the context of chronic pancreatitis.

Previous studies have provided conflicting views of IL-33's role in cancer development. The IL-33/ST2 axis has been shown to promote intestinal tumorigenesis and metastasis of colorectal cancer, suggesting that IL-33 plays a tumor-promoting role in the colon (Ameri *et al.,* 2019; He *et al.,* 2017; Maywald *et al,* 2015; Zhang *et al,* 2017b). However, these findings have been countered by evidence that IL-33 inhibits colorectal tumor growth, suggesting a tumor-suppressing role instead (Chen *et al,* 2018; Malik *et al,* 2016). These studies have focused on the cytokine function of IL-33. Based on our results, we propose a need to distinguish the cellular source of IL-33 (epithelial versus stromal) and its cell-autonomous versus cytokine function across malignancies in which IL-33 has been implicated in order to reconcile the divergent conclusions. Interestingly, a recent report shows that IL-33/TGF-β cytokine loop between tumor-initiating cells and macrophages promotes skin cancer progression (Taniguchi *et al,* 2020). Our findings suggest that nuclear IL-33 can further amplify TGF-β signaling within the tumor-initiating cells. Ultimately, understanding the distinct contributions of nuclear versus secreted IL-33 is key to developing proper therapeutic strategies directed at IL-33 in cancer and inflammatory diseases.

As shown herein, IL-33 induces a tumor-promoting environment in chronic inflammation of skin and pancreas independent of its cytokine function. IL-33 was highly expressed and localized in the nuclei of skin and pancreatic epithelial cells in chronic inflammation. Nuclear IL-33 repressed Smad6 gene expression by blocking the transcription activity of RUNX2, which led to highly phosphorylated SMAD2/3 (p-SMAD2/3) and p-SMAD1/5. TGF-β/SMAD signaling pathway activation was required for IL-33-induced cell proliferation and tumor development. Furthermore, upregulated IL-33 and SMAD signaling were associated with skin cancer, pancreatitis, and pancreatitis-associated PDAC in humans. The present results indicate that nuclear IL-33/SMAD signaling creates a cell-autonomous axis essential for tumor promotion in chronic inflammatory conditions, e.g., of the skin and pancreas, and thus inhibitors of IL-33 can be used to treat cancer and inflammation, and/or to reduce risk of development of tumors, e.g., in subjects with chronic inflammatory conditions.

### Small Molecule Inhibitors of IL-33

As described herein, a number of inhibitors of IL-33 have been identified, including pitavastatin (e.g., pitavastatin calcium), tropisetron, ammonium glycyrrhizinate, ticagrelor, and cetrimonium bromide. Without wishing to be bound by theory, it is believed that pitavastatin (and potentially the other inhibitors) blocks the phosphorylation of IRF3 transcription factor, which is believed to be the transcription factor that induces IL-33 expression in chronic inflammation.

The structures of each of the inhibitors is set forth below.

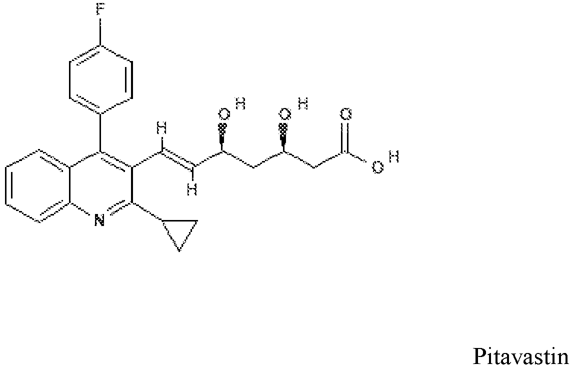

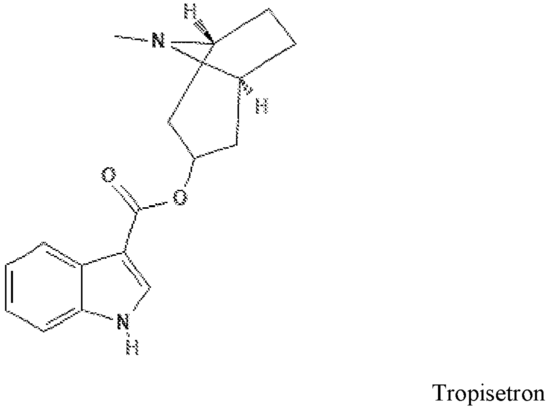

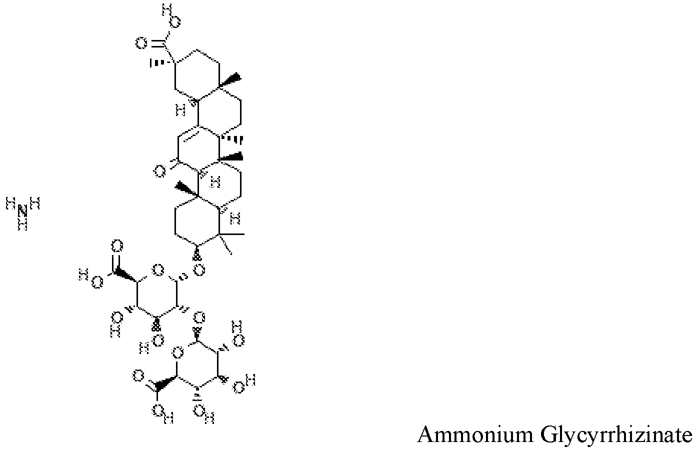

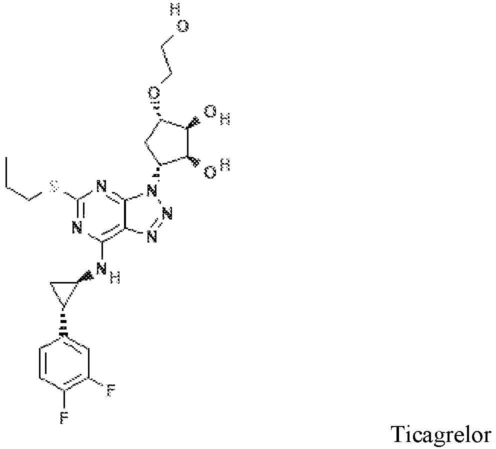

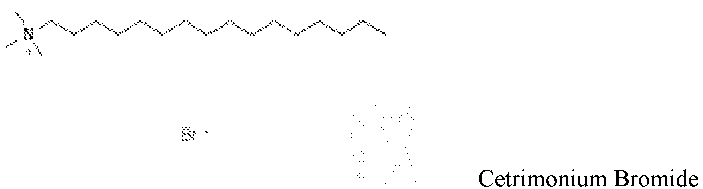

### Methods of Treatment

As shown herein, nuclear IL-33 plays a key role in driving inflammation, fibrosis, and cancer development, e.g., in the context of chronic inflammation. The methods described herein include methods for the treatment of disorders associated with inflammation, fibrosis, cancer, or IL-33 activation. Generally, the methods include administering a therapeutically effective amount of an inhibitor as described herein, to a subject who is in need of, or who has been determined to be in need of, such treatment.

As used in this context, to "treat" means to ameliorate at least one symptom of the disorder. Often, inflammation results in fibrosis and tumorigenesis; thus, a treatment can result in a reduction in fibrosis or tumor burden, or a reducing in risk of developing fibrosis or cancer. Administration of a therapeutically effective amount of a compound described herein for the treatment of a condition associated with inflammation, fibrosis, cancer, or IL-33 activation will result in decreased inflammation, fibrosis, cancer, cancer risk, or IL-33 activation.

### Inflammation

IL-33 strongly induces pro-inflammatory cytokine production, including Th2 cytokines from immune cells, and can promote the pathogenesis of Th2-related disease. The methods described herein include methods for the treatment of disorders associated with abnormal inflammation, e.g., inflammation, e.g., inflammation associated with IL-33 activation, e.g., allergic, fibrotic, infectious, and chronic inflammatory diseases (see, e.g., Cayrol and Girard, Immunol Rev. 2018 Jan;281(1):154-168). In some embodiments, the disorder is a chronic inflammatory condition such as allergic asthma, atopic dermatitis, or inflammatory bowel disease (IBD), allergic contact dermatitis, hepatitis, pancreatitis, or an acute condition such as anaphylaxis. See also, e.g., Pastorelli et al., Mediators of Inflammation, vol. 2013, Article ID 608187, 11 pp (2013); Miller, J Inflamm 8, 22 (2011); Gabryelska et al., Front Immunol. 10: 692 (2019); Chan et al., Front Immunol. 10: 364 (2019); Takatori et al., Front Immunol. 2018 Sep 4;9:2004; Chen et al., Cell Physiol Biochem. 2018;49(1):349-358; and Molofsky et al., Immunity. 2015 Jun 16;42(6):1005-19. In some embodiments, the disorder is organ fibrosis, e.g., fibrosis of skin (e.g., scleroderma, Dupuytren's disease (DD)), hepatic, pancreatic, renal, bowel (e.g., IBD-associated fibrosis), or pulmonary tissues; see, e.g., Izadi et al., Science Advances 04 Dec 2019:Vol. 5, no. 12, eaay0370; Kotsiou et al., Front Immunol. 2018; 9: 2432; Rankin et al., J Immunol 2010; 184:1526-1535; Fanny et al., Front Immunol. 2018; 9: 1476; Milovanovic et al., Immunol Res (2012) 52:89-99. Generally, the methods include administering a therapeutically effective amount of a treatment as described herein, to a subject who is in need of, or who has been determined to be in need of, such treatment. In some embodiments, the methods include detecting a level of IL-33 in the tumor, comparing the level to a reference level, and administering a therapeutically effective amount of a treatment as described herein to a subject who has a level of IL-33 above the reference level. In some embodiments, the methods also include administering an anti-inflammatory agent, e.g., a corticosteroid or non-steroidal anti-inflammatory drug (NSAID).

As used in this context, to "treat" means to ameliorate at least one symptom of the disorder associated with abnormal inflammatory processes. For example, a treatment can result in a reduction in inflammation, fibrosis, symptoms of allergy, or severity of anaphylaxis.

### Cancer

The methods described herein include methods for the treatment of disorders associated with abnormal apoptotic or differentiative processes, e.g., cancer, e.g., cancer associated with IL-33 activation, e.g., in subjects with chronic inflammatory disease. In some embodiments, the disorder is a solid tumor, e.g., breast, prostate, pancreatic, brain, hepatic, lung, renal, skin, ovarian, or colon cancer; see e.g., Liu et al., Oncol Lett. 2021 Jul;22(1):504 (IL-33 in ovarian cancer). Generally, the methods include administering a therapeutically effective amount of a treatment as described herein, to a subject who is in need of, or who has been determined to be in need of, such treatment. In some embodiments, the methods include detecting a level of IL-33 in the tumor, comparing the level to a reference level, and administering a therapeutically effective amount of a treatment as described herein to a subject who has a level of IL-33 above the reference level. In some embodiments, the methods also include administering an immunotherapy, e.g., a checkpoint inhibitor, and/or a standard treatment comprising chemotherapy, radiotherapy, and/or resection.

As used in this context, to "treat" means to ameliorate at least one symptom of the disorder associated with abnormal apoptotic or differentiative processes. For example, a treatment can result in a reduction in tumor size or growth rate, or a reduction in risk of developing a cancer. Administration of a therapeutically effective amount of a compound described herein for the treatment of a condition associated with abnormal apoptotic or differentiative processes will result in a reduction in tumor size or decreased growth rate, a reduction in risk or frequency of reoccurrence, a delay in reoccurrence, a reduction in metastasis, increased survival, and/or decreased morbidity and mortality, *inter alia.* The present methods can also be used for prophylaxis, i.e., to reduce risk of developing cancer, e.g., in a subject who has a chronic inflammatory disease.

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin.

As used herein, the terms "cancer", "hyperproliferative" and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as affecting lung, breast, thyroid, lymphoid, gastrointestinal, and genitourinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. **In** some embodiments, the disease is renal carcinoma or melanoma. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

### Dosage

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a therapeutic compound (i.e., an effective dosage) depends on the therapeutic compounds selected. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the therapeutic compounds described herein can include a single treatment or a series of treatments.

Dosage, toxicity and therapeutic efficacy of the therapeutic compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

### Pharmaceutical Compositions and Methods of Administration

The methods described herein include the use of pharmaceutical compositions comprising or consisting of pitavastatin (e.g., pitavastatin calcium), tropisetron, ammonium glycyrrhizinate, ticagrelor, and/or cetrimonium bromide as an active ingredient.

Pharmaceutical compositions typically include a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions, e.g., for treating cancer: chemotherapy, targeted therapy and immunotherapy including immune checkpoint blockade (e.g., anti PD1, CTLA4, PD-L1, TIGIT, LAG3, TIM3 and Lair1 antibody therapies), CAR T cell and adoptive T cell therapy, innate immunotherapies (e.g., STING and TLR agonist therapies) and oncolytic virus treatment.

Pharmaceutical compositions are typically formulated to be compatible with its intended route of administration. Examples of routes of administration include parenteral, e.g., intravenous, intradermal, subcutaneous, oral, nasal (e.g., inhalation), transdermal (topical), transmucosal, and rectal administration.

Methods of formulating suitable pharmaceutical compositions are known in the art, see, e.g., Remington: The Science and Practice of Pharmacy, 21st ed., 2005; and the books in the series Drugs and the Pharmaceutical Sciences: a Series of Textbooks and Monographs (Dekker, NY). For example, solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent that delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying, which yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of a mist, a fine powder, or an aerosol spray, e.g., from a pressured container or dispenser, e.g., inhaler, insufflator, or nebulizer, that optionally contains a suitable propellant, e.g., a gas such as carbon dioxide. Such methods include those described in U.S. Patent No. 6,468,798. An aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas, can also be used. **In** the case of a pressurized aerosol the dosage unit can be determined by providing a valve to deliver a metered amount. Capsules and cartridges of e.g., gelatin, for use in an inhaler or insufflator may be formulated containing a powder mix of the compound and a suitable powder base such as lactose or starch.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories.

For topical or transdermal administration, the active compounds can be formulated into ointments, salves, gels, lotions, or creams as generally known in the art.

**In** some embodiments, compositions comprising an inhibitor as described herein for topical or transdermal application (e.g., for treatment of skin inflammation, fibrosis, or cancer) can further comprise cosmetically-acceptable carriers or vehicles and any optional components. A number of such cosmetically acceptable carriers, vehicles and optional components are known in the art and include carriers and vehicles suitable for application to skin (e.g., sunscreens, creams, milks, lotions, masks, serums, etc.), see, e.g., U.S. Patent Nos. 6,645,512 and 6,641,824. **In** particular, optional components that may be desirable include, but are not limited to absorbents, anti-acne actives, anti-caking agents, anti-cellulite agents, anti-foaming agents, anti-fungal actives, anti-inflammatory actives, anti-microbial actives, anti-oxidants, antiperspirant/deodorant actives, anti-skin atrophy actives, anti-viral agents, anti-wrinkle actives, artificial tanning agents and accelerators, astringents, barrier repair agents, binders, buffering agents, bulking agents, chelating agents, colorants, dyes, enzymes, essential oils, film formers, flavors, fragrances, humectants, hydrocolloids, light diffusers, nail enamels, opacifying agents, optical brighteners, optical modifiers, particulates, perfumes, pH adjusters, sequestering agents, skin conditioners/moisturizers, skin feel modifiers, skin protectants, skin sensates, skin treating agents, skin exfoliating agents, skin lightening agents, skin soothing and/or healing agents, skin thickeners, sunscreen actives, topical anesthetics, vitamin compounds, and combinations thereof.

The pharmaceutical compositions can also be prepared in the form of suppositories (e.g., with conventional suppository bases such as cocoa butter and other glycerides) or retention enemas for rectal delivery.

In one embodiment, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques, or obtained commercially, e.g., from Alza Corporation and Nova Pharmaceuticals, Inc. Liposomal suspensions (including liposomes targeted to selected cells with monoclonal antibodies to cellular antigens) can also be used as pharmaceutically acceptable carriers. These can be prepared according to methods known to those skilled in the art, for example, as described in U.S. Patent No. 4,522,811.

The pharmaceutical compositions can be included in a container, pack, or dispenser together with instructions for administration.

### EXAMPLES

The invention is further described in the following examples, which do not limit the scope of the invention described in the claims.

### Example 1. Nuclear IL-33/SMAD signaling axis promotes cancer development in chronic inflammation.

Interleukin (IL)-33 cytokine plays a critical role in allergic diseases and cancer. IL-33 also has a nuclear localization signal. However, the nuclear function of IL-33 and its impact on cancer is unknown. Here, we demonstrate that nuclear IL-33-mediated activation of SMAD signaling pathway in epithelial cells is essential for cancer development in chronic inflammation. Using RNA- and ChIP-sequencing, we found that nuclear IL-33 repressed the expression of an inhibitory SMAD, Smad6, by interacting with its transcription factor, RUNX2. IL-33 was highly expressed in the skin and pancreatic epithelial cells in chronic inflammation, leading to a markedly repressed Smad6 expression as well as dramatically upregulated p-SMAD2/3 and p-SMAD1/5 in the epithelial cells. Blocking TGF-β/SMAD signaling attenuated the IL-33-induced cell proliferation in vitro and inhibited IL-33-dependent epidermal hyperplasia and skin cancer development in vivo. IL-33 and SMAD signaling were upregulated in human skin cancer, pancreatitis, and pancreatitis-associated pancreatic cancer. Collectively, our findings reveal that nuclear IL-33/SMAD signaling is a cell-autonomous tumor-promoting axis in chronic inflammation, which can be targeted by small-molecule inhibitors for cancer treatment and prevention.

### Materials and Methods

The following materials and methods were used in Example 1 below.

### Human samples

De-identified slides of formalin-fixed paraffin-embedded human tissue sections were obtained from the Department of Pathology at Massachusetts General Hospital.

### Animal studies

All mice were housed under pathogen-free conditions in an animal facility at Massachusetts General Hospital in accordance with animal care regulations. WT C57BL/6 mice were purchased from the Jackson Laboratory (Bar Harbor, ME). K14-IL33^{tg} mice were purchased from the Transgenic Inc. (Kobe, Japan). IL-33^{KO} mice were a gift from Dr. Marco Colonna and ST2^{KO} mice were a gift from Dr. Peter Nigrovic. All mouse strains are on the C57BL/6 background. Age- and gender-matched mice were used in all experiments.

### Skin chronic inflammation

Four- to six-week-old male and female mice were shaved on their abdomen and sensitized to 50 µL 0.5% 1-Fluoro-2,4-dinitrobenzene (DNFB, Millipore Sigma, St. Louis, MO, catalog no. 42085) dissolved in acetone plus olive oil at a 3:1 ratio (refer to as acetone). Two days after first sensitization, mice were re-sensitized to 50 µL 0.25% DNFB on their abdomen. After 5 days, mice were challenged with 100 µL 0.25% DNFB on their back skin, which was repeated three times per week for 22 days. The skin rash was monitored over the duration of the study.

### Skin carcinogenesis in chronic inflammation

For DMBA/DNFB carcinogenesis protocol (Ameri *et al.,* 2019), four- to six-week-old male and female mice were shaved on their abdomen and sensitized to 50 µL 0.5% DNFB. Two days later, mice were re-sensitized to 50 µL 0.25% DNFB on the abdomen. Five days later, mice were treated with 100 µg 7,12-dimethylbenz(a)anthracene (DMBA, Millipore Sigma, catalog no. D3254) in 200 µL acetone on their back skin. After a week, mice were challenged with 100 µL 0.25% DNFB on their back skin, which was continued three times per week for 30 weeks at the dosage that maintained mild erythematous dermatitis in WT mice. Skin rash and tumor development were monitored and recorded weekly over the duration of the study. For DMBA/TPA carcinogenesis protocol, mice were treated with 6 µg 12-O-tetradecanoylphorbol-13-acetate (TPA, Millipore Sigma, catalog no. P1585) in 200 µL acetone instead of DNFB in DMBA/DNFB protocol without abdominal sensitization.

### Skin treatment with SB431542

To inhibit the TGF-β/SMAD signaling pathway (Mordasky Markell *et al,* 2010), mice were treated with 10 µM SB431542 (Millipore Sigma, catalog no. 301836-41-9) in 200 µL acetone or 200 µL acetone alone to their back skin twice a week. SB431542/acetone treatments were given in between TPA or DNFB doses.

### Colitis-associated colorectal carcinogenesis

Mice were weighed and injected intraperitoneally with 10 mg/kg Azoxymethane (AOM, Millipore Sigma, catalog no. A5486) in 100 µL PBS. One week later, they received a 2% dextran sodium sulfate (DSS, Millipore Sigma, catalog no. 42867) solution in their drinking water for five days. After DSS administration, drinking water was changed to regular water for two weeks. This AOM/DSS protocol was repeated for five cycles. Mice were harvested 20 weeks after initial AOM injection.

### Chronic pancreatitis

Mice were weighed and injected with 50 µg/kg caerulein (BACHEM, Torrance, CA, catalog no. 4030451) in 100 µL of PBS intraperitoneally every hour for 6 hours, three days per week for 4 weeks and mice were harvested.

### Pancreatic cancer in chronic pancreatitis

Mice were anesthetized with isoflurane, and the body and tail of the pancreas were exposed through a right lateral subcostal incision in the abdominal wall and exteriorization of the spleen. 1 mg DMBA in 50 µL PBS with 0.001% of the Tween-20 solution was injected into the tail of the pancreas using a 25G needle (Scarlett *et al.,* 2011). The spleen and pancreas were returned into the abdominal cavity and the peritoneum/muscle layers and skin were sutured and covered with CURAD ointment and petrolatum gauze for a week. One week later, mice were injected with 50 µg/kg caerulein in 100 µL of PBS once a day, three days a week, until harvesting. Mice were monitored for any sign of distress, weight loss or palpable abdominal mass. Mice were harvested 25 weeks after DMBA treatment.

### Cell lines, plasmids and transfection

Pam212 cells (Mouse keratinocyte cell line) were grown at 37°C in DMEM (Thermo Fisher Scientific, Waltham, MA, catalog no.11995065) supplemented with 10% fetal bovine serum (Thermo Fisher Scientific, catalog no. 26140079), 1X penicillin-streptomycin-glutamine (Thermo Fisher Scientific, catalog no. 10378016), 1X MEM non-essential amino acids solution (Thermo Fisher Scientific, catalog no. 11140050), 1X HEPES (Thermo Fisher Scientific, catalog no. 15630080) and 0.1% 2-Mercaptoethanol (Thermo Fisher Scientific, catalog no. 21985023). PyMT cell lines (derived from a primary breast tumor of MMTV-PyMT^{tg} mouse on a C57BL/6 background) were grown at 37 °C in RPMI Medium 1640 (Thermo Fisher Scientific, catalog no. 11875093) supplemented with 10% fetal bovine serum, 1X penicillin-streptomycin-glutamine and 0.1% 2-Mercaptoethanol. cDNAs for mouse IL-33 full length and IL-33 cytokine domain were subcloned in pcDNA3.1-HA vector. Transfections of plasmids or Poly (I:C) (Invivogen, San Diego, CA, catalog no. tlrl-pic) in Pam212 cells were performed using Lipofectamine 2000 (Thermo Fisher Scientific, catalog no. 11668019) according to the manufacturer's instructions.

### Western blot

Cell lysates were prepared in LIPA buffer (Thermo Fisher Scientific, catalog no. 89900) consisting of 1X protease inhibitor cocktail, EDTA-free (Thermo Fisher Scientific, catalog no. A32955). Mice tissues were meshed and lysed by 0.1% TWEEN-20 (Millipore Sigma, catalog no. P1379) in PBS. Mice tissues were frozen by liquid nitrogen and thaw by incubation at 37 °C for further lysis. Cell lysates were sonicated for 20 sec, and then they were centrifugated at 13,200 rpm. After checking the protein concentration of each sample, the same amount of proteins was loaded into Mini-PROTEIN TGX^{™} Gels (BIO-RAD, Hercules, CA, catalog no.456-1083 or 456-1086) with 1X Tris/Glycine/SDS buffer (BIO-RAD, catalog no.1610732). A few minutes later (according to protein size), they were transferred to Immobilon-P membrane (Millipore Sigma, catalog no. IPVH00010) with Transfer buffer (Boston Bioproducts, Ashland, MA, catalog no. BP-190). Then, they were incubated 3% bovine serum albumin (Thermo Fisher Scientific, catalog no. BP1600) or 5% Skim milk (BD biosciences, San Jose, CA, catalog no. 232100) in 1X Tris-Buffered Saline (Boston Bioproducts, catalog no. BM301X) containing 0.1% TWEEN, called TBS-T for 30 min. After the membrane was washed with TBS-T, they were subjected to immunoblot with proper antibodies overnight at 4 °C. The following day, they were incubated with proper secondary antibodies and were developed with Pierce ECL Western blotting substrate kit (Thermo Fisher Scientific, catalog no. 32106). First and secondary antibodies are found in Table A.

### Western blot quantification

Western blot bands were quantified with cSeries Capture software (Azure biosystem, Dublin, CA, catalog no. Azure 600). Each band quantity was calculated by measuring band intensity minus the background. Total proteins were normalized based on GAPDH levels. p-SMAD2/3 and p-SMAD1/5 were normalized based on endogenous SMAD2/3 and SMAD1 levels, respectively. Pictures of full western blot gels are provided as a source data file.

### Immunoprecipitation

Cell lysates were prepared in buffer-A consisting of 50 mM Tris-HCL (pH 8.0), 150 mM NaCl, 0.5% NP-40 and 1X protease inhibitor cocktail. After confirming the concentration of total proteins, the cell lysates were incubated with IL-33 (Nessy-1) overnight at 4 °C and incubated with 30 µL of 50% slurry of protein A-conjugated Sepharose (Cell Signaling Technology, Danvers, MA, catalog no. 9863S) for the next 2 hours. The samples were precipitated, washed three times with buffer-A containing 300 mM NaCl. Samples were added to 25 µL SDS sampling buffer (Boston Bioproducts, catalog no. BP-111R) and boiled at 95°C for 10 min. Samples were subjected to SDS-polyacrylamide gel electrophoresis followed by immunoblot analysis.

### RNA-Seq

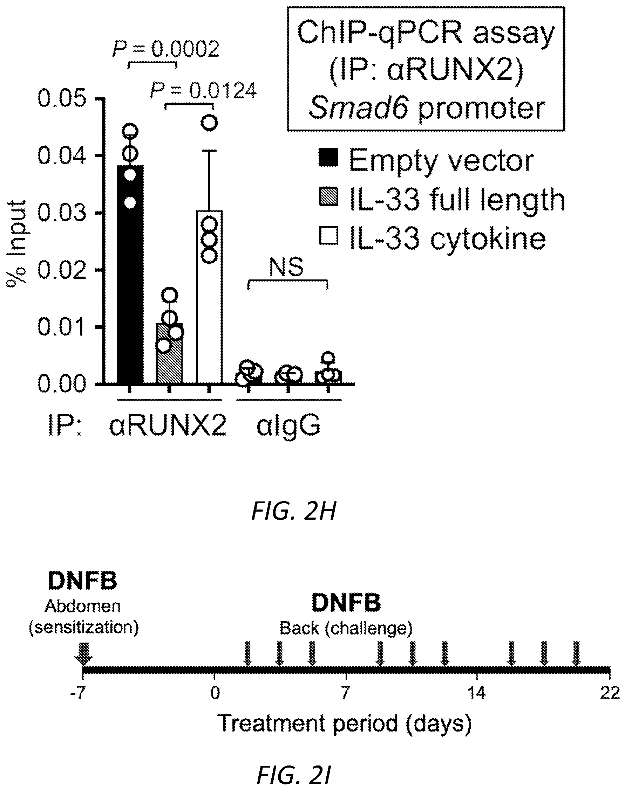

IL-33^{KO} and WT mice were treated with DNFB-induced skin inflammation protocol (Fig 2I) and epidermis was isolated from the back skin. RNA was extracted using an RNeasy micro kit (Qiagen, Hilden, Germany, catalog no. 74004) and quantified using Agilent Bioanalyzer 2100 (Agilent, Santa Clara, CA). Libraries were prepared by Novogene (Sacramento, CA) using the NEBNext Ultra RNA Library Prep kit for Illumina (New England Biolabs, Ipswich, MA, catalog no. E7770). Sequencing was performed by Novogene using the Illumina NovaSeq6000 System. Reads were aligned to the mouse reference genome (mm10) using STAR. Differential expression analysis was performed by Novogene using the DESeq2R package. Each group includes three mice. Original data are available in the NCBI Gene Expression Omnibus (GEO) with accession number GSE149579 (RNA-Seq).

### ChIP-qPCR assay

Pam212 cells that had been transfected with plasmids were fixed in 1% formaldehyde (Millipore Sigma, catalog no. F8775) for 10 min and were washed by cold PBS. Cells were lysed with buffer contained with 2.5% of glycerol, 50 mM HEPES (pH 7.5), 150 mM NaCl, 0.5 mM EDTA, 0.5% NP-40 and 0.25% Triton X-100. After centrifugation at 2,000 rpm, cell lysates were resuspended in buffer A contained with 1 mM Tris/HCl (pH 7.9), 20 mM NaCl and 0.5 mM EDTA and incubated at room temperature for 10 min. Following the second centrifugation at 2,000 rpm, cells were sonicated in sonication buffer containing 10 mM HEPES, 1 mM EDTA and 0.5% SDS for 30 min to achieve chromatin fragmentation. Following centrifugation at 13,000 rpm, proteins were immunoprecipitated with anti-RUNX2 or anti-IL-33 antibody at 4 °C overnight. The next day, the samples were incubated with 30 µL 50% slurry of ChIP-Grade Protein G agarose beads (Cell Signaling Technology, catalog no. 9007S) for 2 hours. Samples were precipitated, washed three times with wash buffer containing 20 mM Tris/HCl (pH 7.9), 10 mM NaCl, 2 mM EDTA, 0.1% SDS and Triton X-100. After the last wash step, samples were eluted with 100 µL of TE buffer contained with 100 mM Tris/HCl (pH 8), 1mM EDTA and 1% SDS three times. De-crosslinking was performed by overnight incubation with 15 µL of 3 M NaCl at 65°C. Precipitated DNA was purified by ChIP DNA Clean & Concentrator (ZYMO research, Irvine, CA, catalog no. D5205) and subjected to quantitative PCR.

### ChIP-Seq

Mouse Pam212 cell line was transfected with HA-IL-33 full length or HA-IL-33 cytokine domain or HA-empty vector. Transfections of these vectors were performed using Lipofectamine 2000 according to the manufacturer's instructions. After 24 hours, each cell lysate underwent ChIP assay using anti-HA antibody and eluted DNA was purified by ChIP DNA Clean & Concentrator to obtain 50 ng DNA for each sample. Sequencing was performed by Novogene using the Illumina NovaSeq6000 System. For the raw data which passed the quality control, the adapter sequences and low-quality bases were trimmed off to get clean data. The MACS2 software was used to predict the fragment sizes of IP experiments. The fragment sizes were used in the peak calling. MACS2 software (threshold q value =0.05) was used to perform peak calling and find the peak-related genes (Zhang *et al,* 2008). Original data are available in the NCBI Gene Expression Omnibus (GEO) with accession number GSE149579 (ChIP-Seq).

### Histology, immunohistochemistry and immunofluorescence

Tissue samples were collected and fixed in 4% paraformaldehyde (Millipore Sigma, catalog no. P6148) overnight at 4°C. Next, tissues were dehydrated in ethanol, processed and embedded in paraffin. 5 µm sections paraffin-embedded tissues were placed on slides, deparaffinized and stained with H&E. For immunohistochemistry, antigen retrieval was performed in 500 µL of antigen unmasking solution (VECTOR Laboratories, Burlingame, CA, catalog no. H3300) diluted in 50 mL water using Cuisinart pressure cooker for 20 min at high pressure. Slides were washed three times for three minutes each in 1X TBS with 0.025% Triton X-100. Sections were blocked with 1% bovine serum albumin (Thermo Fisher Scientific, catalog no. BP1600) and 5% goat serum (Millipore Sigma, catalog no. G9023) for 1 hour. Slides were stained overnight at 4°C with a primary antibody diluted in TBS containing 1% BSA (Table A). The following day, slides were washed as above and incubated in 100 µL biotinylated secondary antibody (VECTOR Laboratories, catalog no. PK-6200) for 30 min. After washing, slides were incubated with 100 µL mixture of reagent A and B from VECTASTAIN Elite ABC universal kit Peroxidase (VECTOR Laboratories, catalog no. PK-6200) for 30 min. After washing, slides were incubated with 100 µL ImmPACT DAB chromogen stating (VECTOR Laboratories, catalog no. SK-4105) for a few minutes. Finally, slides were dehydrated in ethanol and xylene and mounted with a coverslip using 3 drops of mounting media. For immunofluorescence staining, rehydrated tissue sections were permeated with 1XPBS supplemented with 0.2% Triton X-100 for 5 min. Antigen retrieval was performed similar to immunohistochemistry. Slides were washed three times for three minutes each in 1XPBS with 0.1% TWEEN20. Sections were blocked with 1% bovine serum albumin and 5% goat serum for 1 hour. The slides were stained overnight at 4°C with primary antibodies (Table A). The following day, slides were washed as above and incubated for two hours at room temperature with secondary antibodies conjugated to fluorochromes (Table A). Next, slides were incubated with 1:2000 DAPI (Thermo Fisher Scientific, catalog no. D3571) for five minutes at room temperature, then washed as above. Slides were mounted with Prolong Gold Antifade Reagent (Thermo Fisher Scientific, catalog no. P36930). The number of positive cells was counted in randomly selected high power field (HPF, 200X magnification) images in a blinded manner by a trained investigator. Clinical samples were reviewed by a pathologist.

### Quantitative PCR

Mouse dorsal skin samples were homogenized and lysed in RLT lysis solution (QIAGEN, catalog no. 79216)/0.1% MeOH using Mini-BeadBeater-8 (BioSpec Products, Inc., Bartlesville, OK). Total RNA was extracted using an RNeasy micro kit and quantified using NanoDrop ND-1100 (NanoDrop Technologies, Wilmington, DE). cDNA samples were synthesized from 1 µg of total RNA using Invitrogen SuperScripts III Reverse Transcriptase (Thermo Fisher Scientific, catalog no. 18080085). Expression levels of all cDNA samples were determined with LightCycler 480 system (Roche Molecular Systems Inc., Pleasanton, CA) using iTaq Universal SYBR green supermix (Bio-Rad Laboratories, Hercules, CA, catalog no. 1725121) or TaqMan Universal Master Mix II (Thermo Fisher Scientific, catalog no. 44-400-40). Primer sequences for SYBR green and Taqman assays are listed in Table A. Quantitative real-time PCR for SYBR green analyses were performed in a final reaction volume of 25 µL consisting of 5 µL of cDNA of the respective sample and 12.5 µL of SYBR green master mix mixed with the corresponding primers (2 µM) for each gene. The TaqMan analysis was performed in a final reaction of 10 µL with 4.5 µL cDNA and 5.5 µL TaqMan master mix and corresponding primers (20 µM). All assays were conducted in triplicate and normalized to *Gapdh* expression.

### PCR

DNA was isolated from mouse tissue using KAPA Express Extract buffer and KAPA Express extract Enzyme from Mouse genotyping kit (Kapa Biosystems Inc, Wilmington, MA, catalog no. KK7302). After tissue lysis, PCR was performed with a 2X KAPA2G Fast genotyping mix from the same kit using primers listed in Table A.

### Proliferation assay

Pam212 cells were transfected with siIl33 (QIAGEN, catalog no. SI00870443) or siCon (QIAGEN, catalog no. 1022076) using Lipofectamine RNAiMax Transfection reagent (Thermo Fisher Scientific, catalog no. 13778075) according to the manufacturer's instructions. The following day, cells were divided into 96 well plates with a serial dilution of cells. The next day, the cells were treated with 5 nM of TGF-β (Cell Signaling Technology, catalog no. 5231LF) or 10 µM of SB431542 for 3 hours. After incubation, the proliferation assay was performed by Cell Titer 96 Non-radioactive Cell proliferation assay (Promega, Madison, WI, catalog no. G4001) according to the manufacturer's instructions. O.D was recorded as the measure of cell proliferation in each well. Absorbance (O.D) reading is directly proportional to the cell proliferation in each well.

### Nuclear extraction

Pam212 cell line was transfected with poly (I:C) using Lipofectamine 2000 according to the manufacturer's instructions. After 8 hours, cells were lysed using Nuclear Extraction Kit (Novus Biologicals, Littleton, CO, catalog no. NBP2-29447). Briefly, cells were washed with 1XPBS supplemented with PMSF (Novus biologicals, catalog no. KC-407). Cells were centrifuged for 5 min at 1,000 rpm. Cell pellets were resuspended in 1X cold hypotonic buffer (Novus biologicals, catalog no. KC-401) and incubated on ice for 15 min. After adding 10 µL of the 10% detergent solution (Novus biologicals, catalog no. KC-403), cell lysates were centrifuged 10 min at 14,000 rpm. The supernatants were kept as a cytoplasmic fraction and the cell pellets were resuspended in 100 µL nuclear lysis buffer (Novus biologicals, catalog no. KC-402). Lysates from the pellets were sonicated for a few seconds and incubated for 10 min at 4°C. Then, lysates were centrifuged at 14,000 rpm for 10 min. The supernatants were kept as the nuclear fraction.

### Statistical analysis

Unpaired *t*-test was used as the test of significance for tumor volume, counts, epidermal thickness, count of nuclear protein staining, RNA and protein expression levels and other quantitative measurements. The log-rank test was used as the test of significance for time to tumor onset outcomes. A paired *t*-test was used for the comparison of IL-33⁺ and pSMAD2/3⁺ cell counts and *t*-test for the Pearson correlation coefficient was used to determine significance in the correlation between IL-33⁺ and pSMAD2/3⁺ counts across matched human pancreatic samples. *P-value <* 0.05 is considered significant. Bar graphs show mean + SD.

### Study approval

Massachusetts General Hospital IRBs approved the analysis of de-identified clinical samples. Massachusetts General Hospital IACUC approved animal studies.

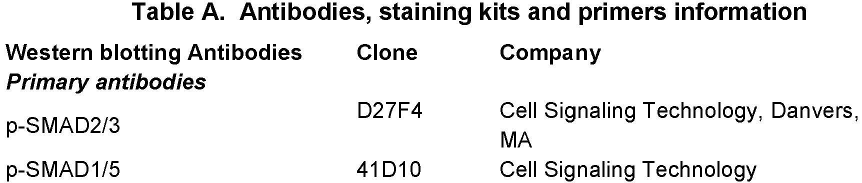

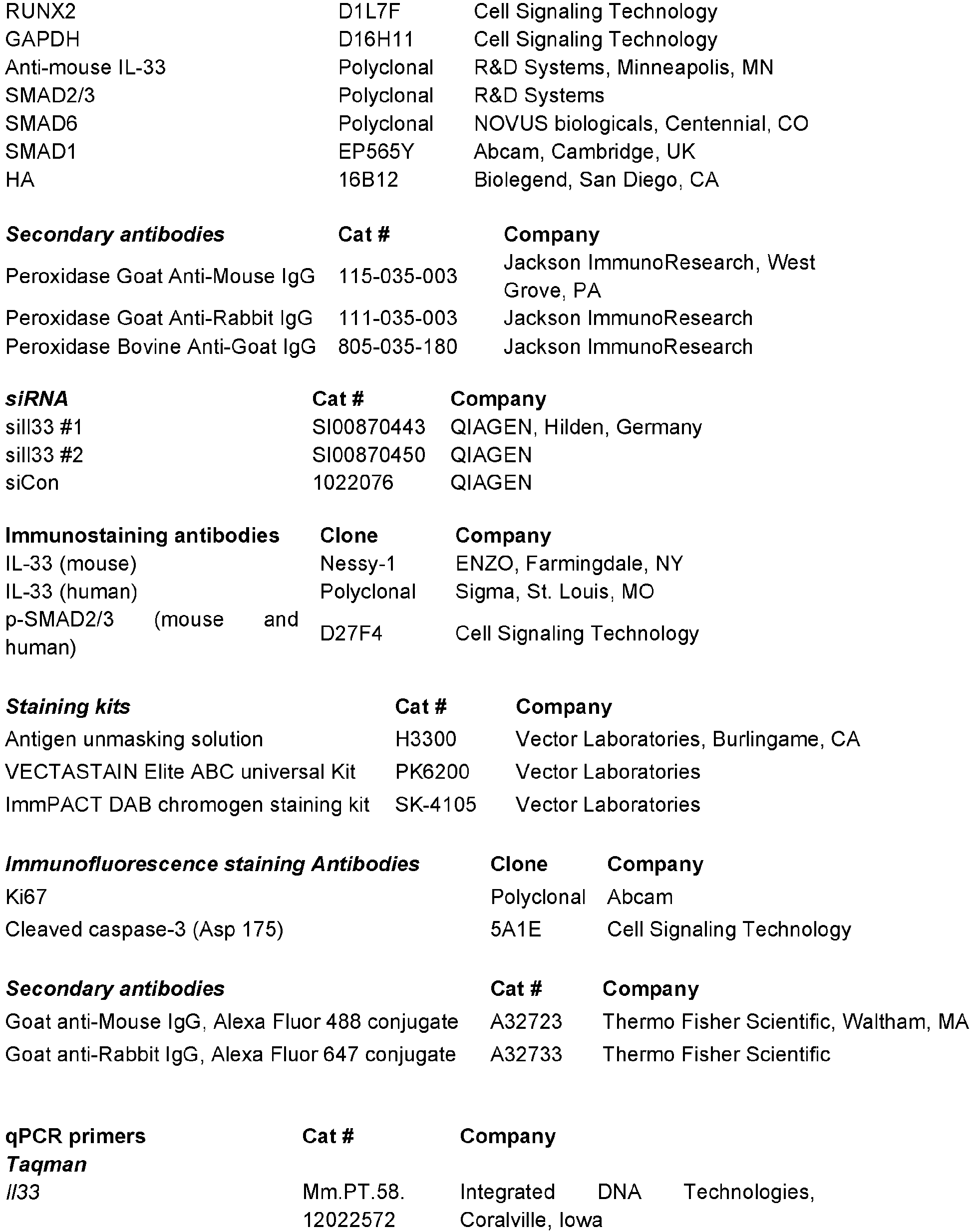

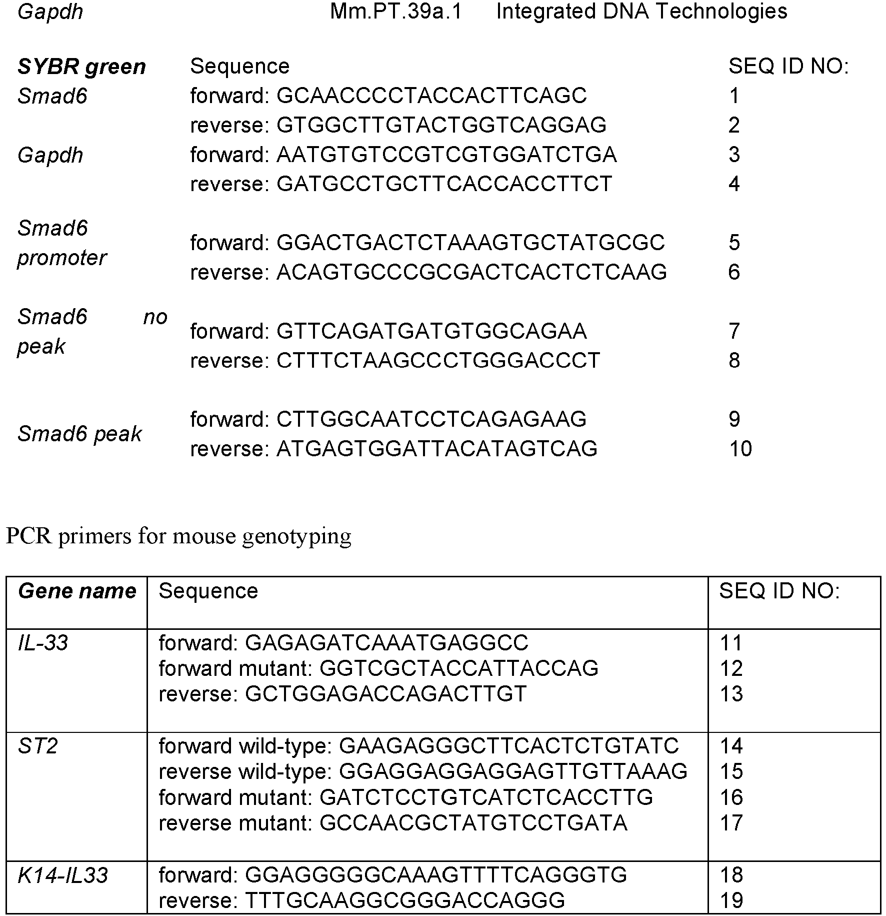

### Example 1.1. IL-33 promotes tumor development in chronic skin inflammation independent of ST2

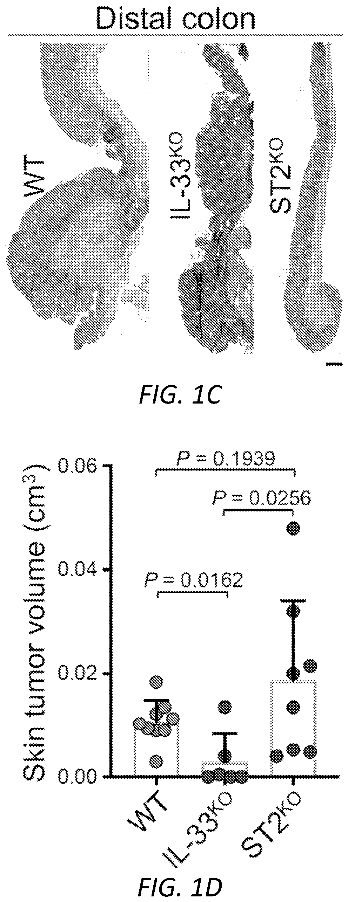

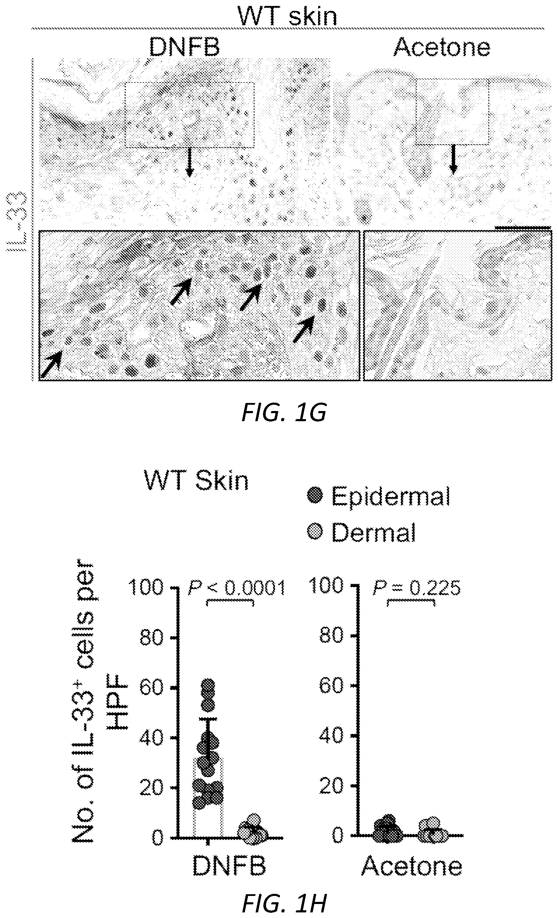

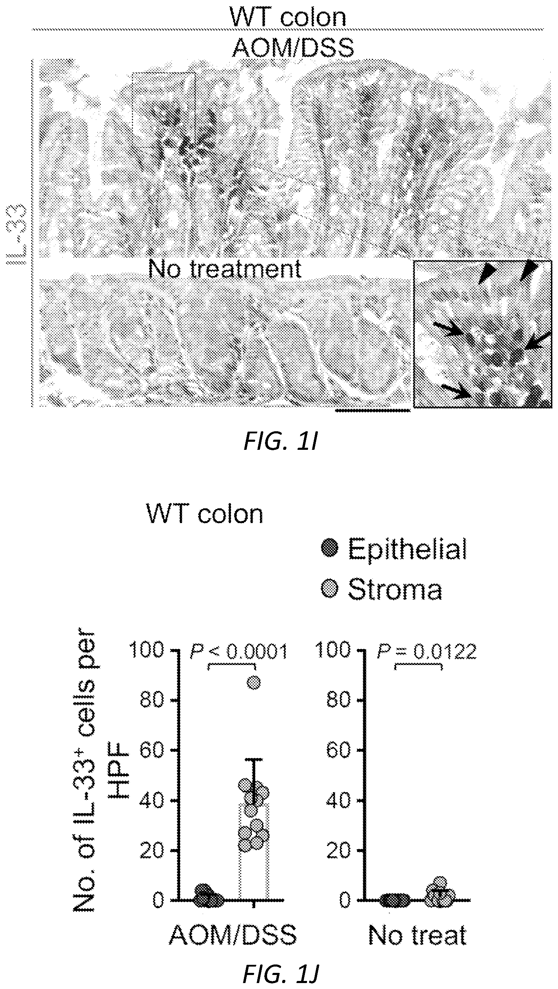

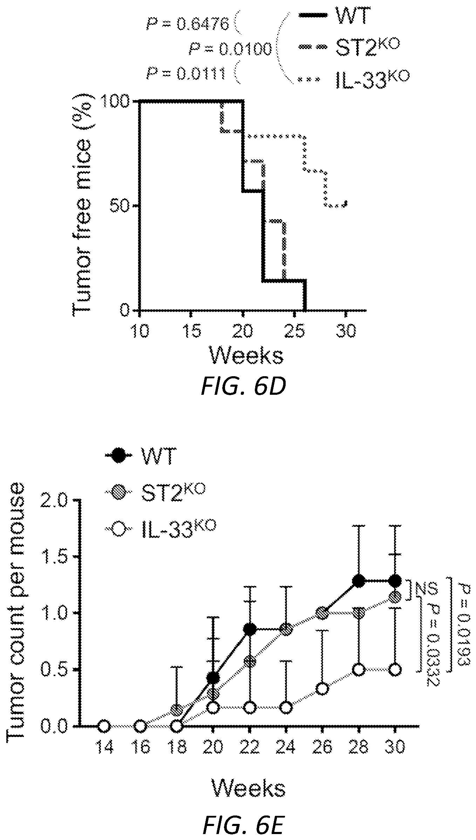

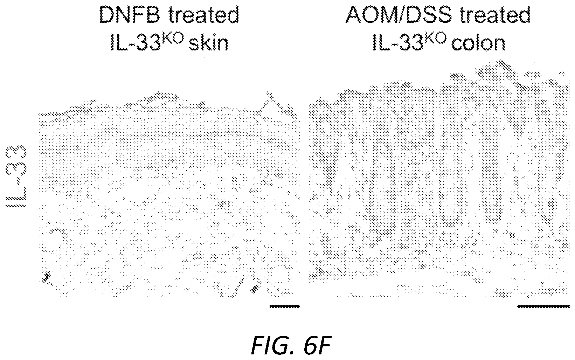

To elucidate the impact of IL-33 on cancer development in chronic inflammation, we subjected *Il33* knockout (IL-33^{*tm1b*/*tm1b*} or IL-33^{KO}), *Il1rl1* knockout (ST2^{KO}) and wild-type (WT) mice to an established model of colitis-induced colorectal cancer. Mice received five treatment cycles of intraperitoneal injection with azoxymethane (AOM) followed by a colitis-causing agent, dextran sodium sulfate (DSS), added to the drinking water over 5 days (Fig 6A). IL-33^{KO} and ST2^{KO} mice developed fewer and smaller colorectal tumors compared with WT mice (Fig 1A and B). Moreover, crypt architecture was better preserved in both IL-33^{KO} and ST2^{KO} compared with the WT colon (Fig 1C). Colitis-induced shortening of the colon length was significantly attenuated in ST2^{KO} compared with WT mice (Fig 6B). Next, we examined the impact of IL-33 on tumor promotion in the context of chronic skin inflammation (Ameri *et al.,* 2019). IL-33^{KO} ST2^{KO}, and WT mice were treated with 7,12-dimethylbenz(a)anthracene (DMBA, a carcinogen) once followed by 2,4-Dinitro-1-fluorobenzene (DNFB, a contact allergen) in acetone on the back skin three times a week for 30 weeks (Fig 6C) (Ameri *et al.,* 2019). Although IL-33^{KO} mice were protected from DMBA-DNFB induced skin carcinogenesis (Ameri *et al.,* 2019), ST2^{KO} mice developed large skin tumors similar to WT mice (Fig 1D-F). In addition, there was no difference between ST2^{KO} and WT mice in skin tumor onset and the number of tumors that developed over time, while IL-33^{KO} showed significantly delayed tumor onset and fewer tumors compared with ST2^{KO} and WT mice over time (Figs 6D and E). To determine the reason for the discordant skin cancer yet concordant colorectal cancer outcomes between IL-33^{KO} and ST2^{KO} mice, we examined the cellular source of IL-33 in the inflamed skin versus colon tissue. Topical DNFB treatment led to significantly elevated IL-33 levels localized in the nuclei of keratinocytes, which are cancer forming cells in the skin (Fig 1G and H, and Fig 6F). In stark contrast, IL-33 was mostly overexpressed by stromal cells, but not colon epithelial cells, in AOM/DSS-induced colitis (Fig 1I and J, and Fig 6F). These findings suggest that IL-33 acts as a nuclear protein in skin cancer development independent of its receptor, ST2, while it acts as a cytokine to promote colorectal cancer in an ST2-dependent manner.

### Example 1.2. Nuclear IL-33 suppresses Smad6 expression via interaction with RUNX2

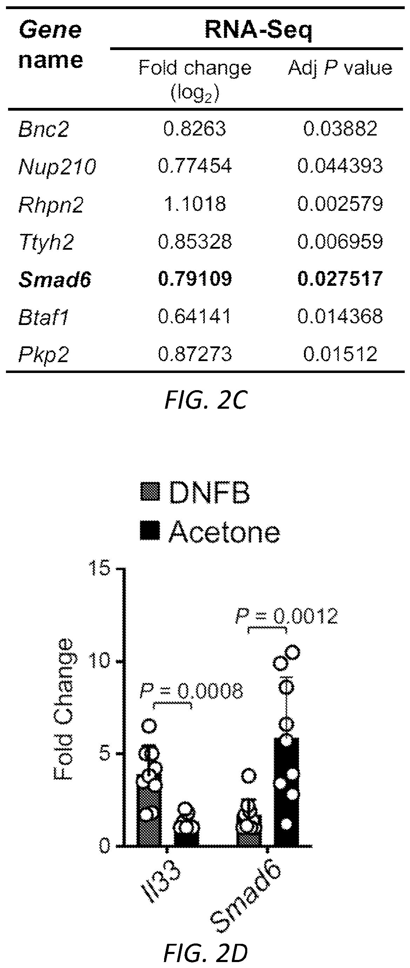

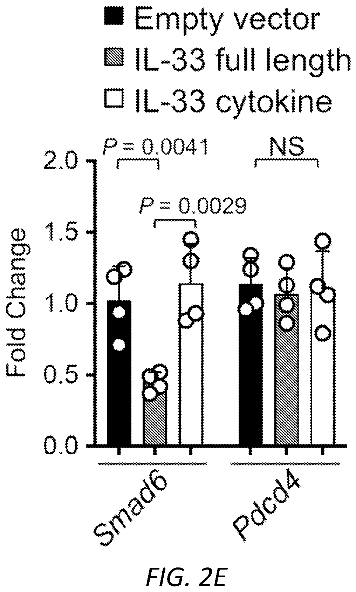

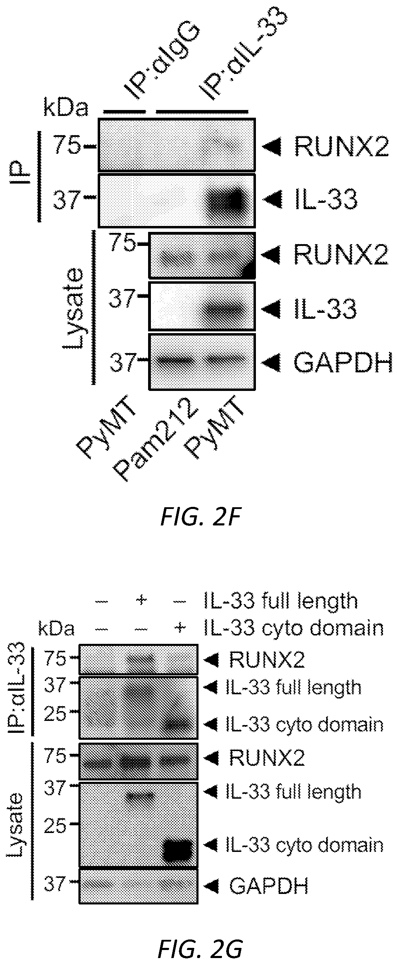

To investigate the nuclear function of IL-33 in chronic inflammation, we performed RNA-sequencing on the epidermal keratinocytes isolated from the back skin of IL-33^{KO} and WT mice treated with DNFB for 22 days (FIG. 2I) (Ameri *et al.,* 2019). 203 genes showed differential expressions in IL-33^{KO} compared with WT epidermal keratinocytes, which belonged to several developmental pathways (Fig 2A). Interestingly, IL-33^{KO} showed negative regulation of the TGF-β/BMP signaling pathway and restricted SMAD proteins phosphorylation by Gene Set Enrichment Analysis (GSEA). To further examine the nuclear function of IL-33, we generated IL-33 full length and IL-33 cytokine domain only constructs (Fig 2B). IL-33 full length localized into the nucleus while IL-33 cytokine domain localized in the cytoplasm of a mouse keratinocyte cell line (Pam212). Chromatin immunoprecipitation (ChIP) sequencing identified 2455 genes with differential peaks in IL-33 full length compared with IL-33 cytokine domain expressing Pam212 cells (Fig 2B). By cross comparing the RNA-Seq and ChIP-Seq data, we narrowed down the nuclear IL-33 candidate targets to 7 genes, all of which showed increased expression upon IL-33 loss in the skin keratinocytes (Fig 2B and C). Among these genes, we focused on *Smad6* because the DNFB-treated IL-33^{KO} epidermis showed downregulation of the SMAD signaling pathway compared with WT by GSEA. IL-33 full length, but not IL-33 cytokine domain, bound to *Smad6* regulatory region. To verify that *Smad6* was a target of IL-33, we performed qPCR on the skin of WT mice treated with DNFB versus acetone (carrier control). *Il33* was highly increased and *Smad6* was markedly decreased in DNFB-treated skin compared with acetone-treated controls (Fig 2D). Importantly, the IL-33 full length, but not cytokine domain, suppressed *Smad6* expression in Pam212 cells compared to HA-empty vector control (Fig 2E). Neither IL-33 full length nor cytokine domain impacted the expression of a control gene, *Pdcd4,* in Pam212 cells (Fig 2E). To determine the mechanism by which IL-33 repressed *Smad6* expression, we examined whether nuclear IL-33 interacted with the *Smad6* transcription factor, RUNX2 (Wang *et al,* 2007). Immunoprecipitation of endogenous nuclear IL-33, which was highly expressed in a breast cancer cell line (PyMT), revealed a strong binding between IL-33 and endogenous RUNX2 (Fig 2F). Overexpression of IL-33 full length or cytokine domain in Pam212 cells revealed that IL-33 full length, but not cytokine domain, bound to RUNX2 (Fig 2G). Furthermore, IL-33 full length, compared with IL-33 cytokine domain, decreased the binding of RUNX2 to the *Smad6* promoter region (Fig 2H). Consistent with these findings, RUNX2 target genes were highly expressed in IL-33^{KO} compared with WT epidermis treated with DNFB. These results indicate that nuclear IL-33 regulates *Smad6* expression via its interaction with RUNX2.

### Example 1.3. Nuclear IL-33 induces epithelial cell proliferation via TGF-β/SMAD signaling pathway

As SMAD6 is an inhibitory SMAD protein, we examined whether IL-33 can suppress the TGF-β/SMAD signaling pathway. IL-33 induction in DNFB-treated WT skin (Fig 1G, H and 2D) was accompanied by a reduction in SMAD6 protein and a marked increase in phosphorylated SMAD2/3 (p-SMAD2/3) and p-SMAD1/5 compared with acetone-treated WT skin (Fig 3A). Furthermore, SMAD6 levels were restored and p-SMAD2/3 and p-SMAD1/5 were greatly reduced in DNFB-treated IL-33^{KO} compared with WT skin. In contrast, no IL-33-mediated regulation of SMAD proteins was observed in the AOM/DSS-treated colon of IL-33^{KO} compared with WT mice. These findings demonstrate that nuclear IL-33 represses *Smad6* expression and activates SMAD signaling pathway when expressed by the epithelial cells in inflamed tissue.

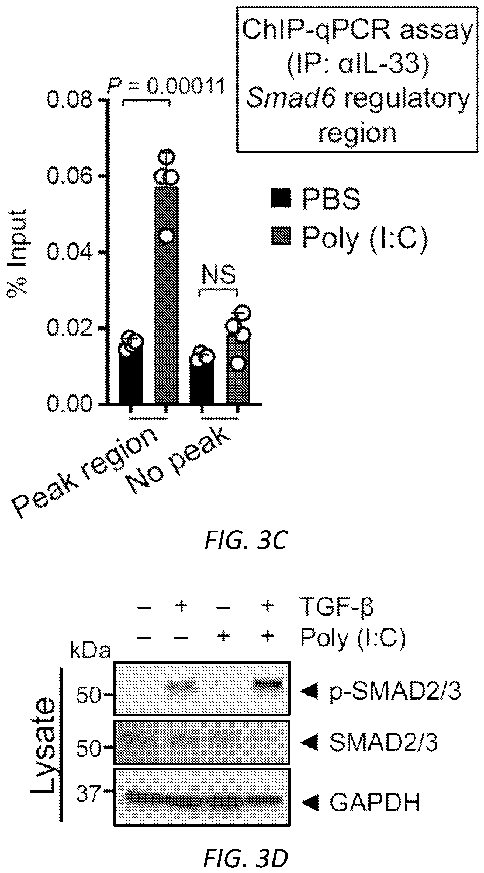

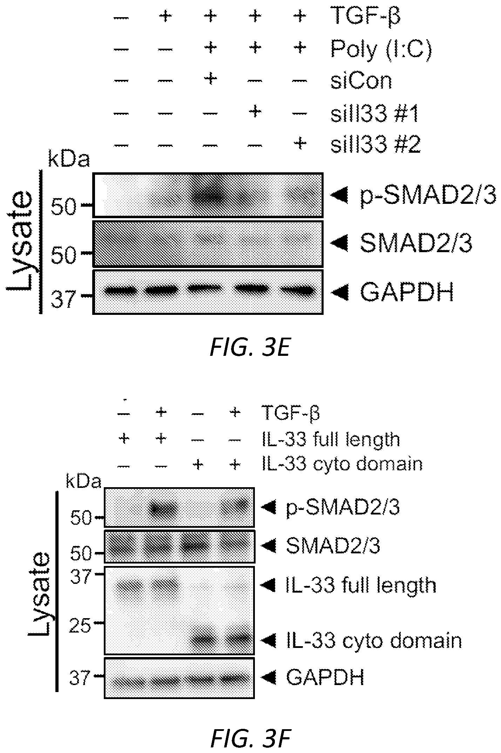

Next, we examined whether IL-33 can directly regulate TGF-β/SMAD signaling within the epithelial cells using polyinosinic-polycytidylic acid (poly (I:C)), a TLR3 agonist that can induce IL-33 and elicit an inflammatory response in epithelial cells (Natarajan *et al,* 2016; Polumuri *et al,* 2012). Poly (I:C) treatment led to *Il33* induction and concomitant *Smad6* repression in Pam212 cells (Fig 3B). Poly (I:C) treatment led to increased IL-33 protein levels in the nuclei of Pam212 cells. Poly (I:C)-induced endogenous IL-33 bound specifically to *Smad6* gene regulatory region corresponding to a peak in our ChIP-Seq results (Fig 3C). We tested the impact of endogenous IL-33 on RUNX2 binding to *Smad6* promoter using siRNA against IL-33 (siIl33) to knock down IL-33 levels in Pam212 cells. RUNX2 binding to *Smad6* promoter was increased after IL-33 knockdown. In contrast, IL-33 induction by poly (I:C) led to a markedly reduced RUNX2 binding to *Smad6* promoter, which was partially rescued by IL-33 knockdown in poly (I:C)-treated cells. Accordingly, poly (I:C) highly augmented the effect of TGF-β treatment on Pam212 cells as shown by increased p-SMAD2/3 levels even though endogenous SMAD2/3 levels remained unchanged (Fig 3D). Importantly, poly (I:C)-induced p-SMAD2/3 upregulation was reversed when IL-33 was knocked down using siIl33 (Fig 3E). The overexpression of IL-33 full length, but not cytokine domain, in TGF-β-treated Pam212 cells increased p-SMAD2/3 levels (Fig 3F). These data demonstrate that nuclear IL-33 promotes TGF-β/SMAD signaling within the epithelial cells.

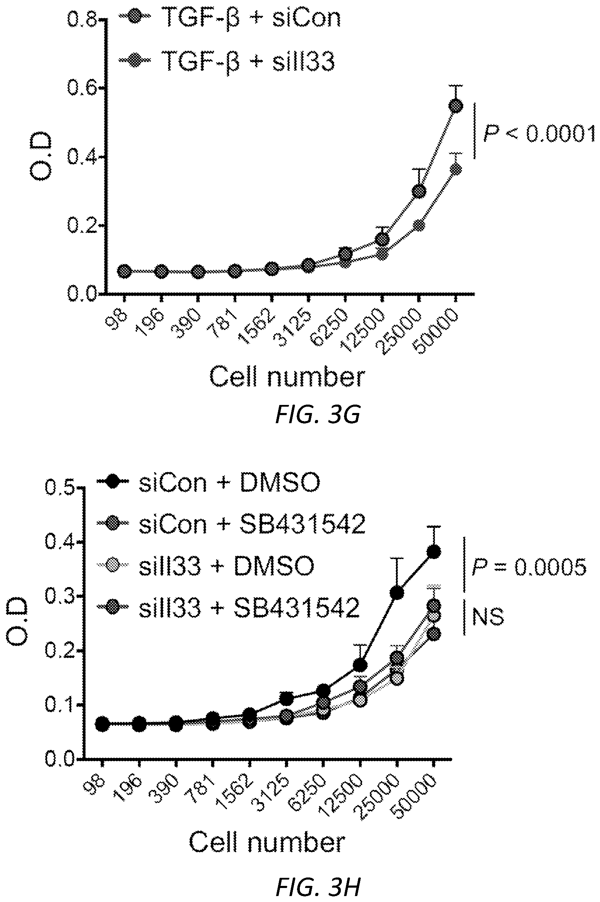

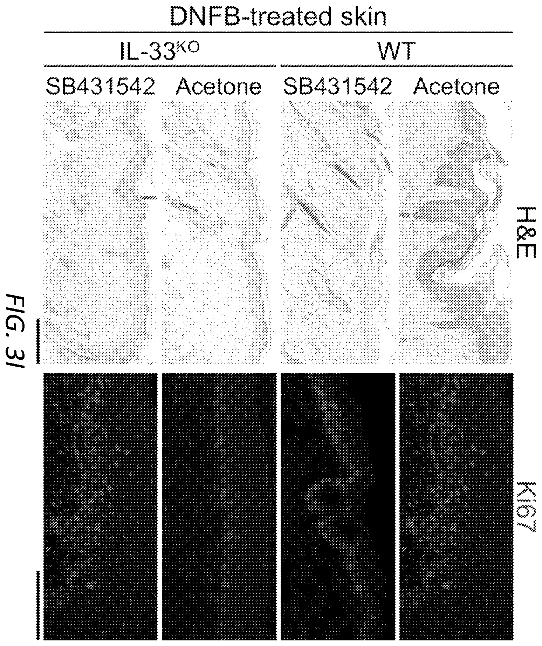

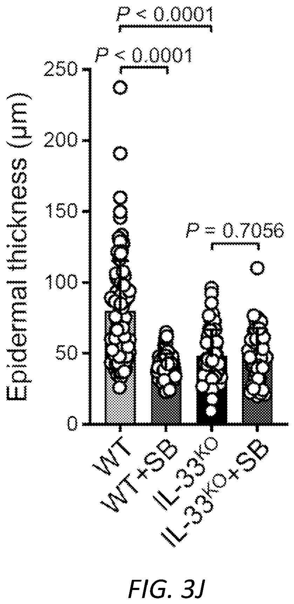

TGF-β/SMAD signaling pathway can promote cell proliferation (Zhang *et al,* 2017a). Therefore, we examined whether IL-33 regulates the TGF-β/SMAD signaling effect on epithelial cell proliferation. Knocking down the endogenous IL-33 levels in TGF-β-treated Pam212 cells resulted in a significant reduction in cell proliferation compared with TGF-β plus control siRNA-treated group (Fig 3G). Endogenous TGF-β expression in Pam212 cells was not affected by knocking down IL-33. Treatment of Pam212 cells with SB431542, an inhibitor of TGF-β receptor (Hjelmeland *et al,* 2004; James *et al,* 2010), significantly reduced Ki67⁺ proliferating cells. SB431542 treatment reduced Pam212 cell proliferation to the same degree as knocking down IL-33 (Fig 3H). Importantly, knocking down IL-33 in Pam212 cells that were treated with SB431542 did not further reduce cell proliferation (Fig 3H). These results indicate that IL-33 regulates cell proliferation via TGF-β/SMAD signaling pathway. To investigate the interplay of IL-33 and TGF-β/SMAD signaling in promoting cell proliferation *in vivo,* WT and IL-33^{KO} mice were treated with DNFB plus either SB431542 (test) or acetone (control) for 22 days. DNFB-induced epidermal hyperplasia and keratinocyte proliferation in WT mice was significantly attenuated in IL-33^{KO} mice (Fig 3I and J) (Ameri *et al.,* 2019). However, no change in epidermal keratinocyte apoptosis was observed. SB431542 treatment markedly decreased the epidermal hyperplasia and keratinocyte proliferation in DNFB-treated WT mice (Fig 3I and J). However, SB431542 treatment had no impact on the epidermal hyperplasia or keratinocyte proliferation in DNFB-treated IL-33^{KO} mice (Fig 3I and J). These findings demonstrate that nuclear IL-33 promotes epithelial cell proliferation via TGF-β/SMAD signaling pathway.

### Example 1.4. Nuclear IL-33/SMAD signaling pathway promotes skin carcinogenesis

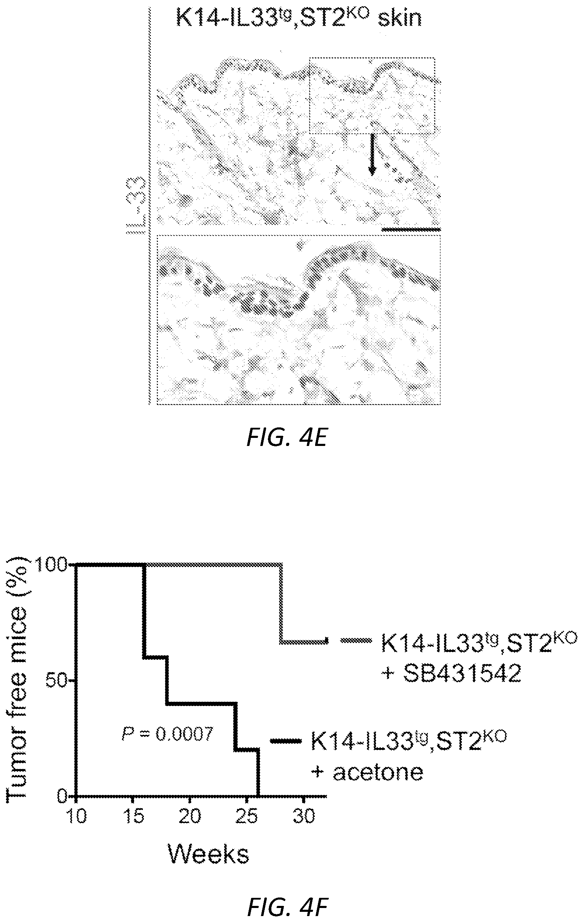

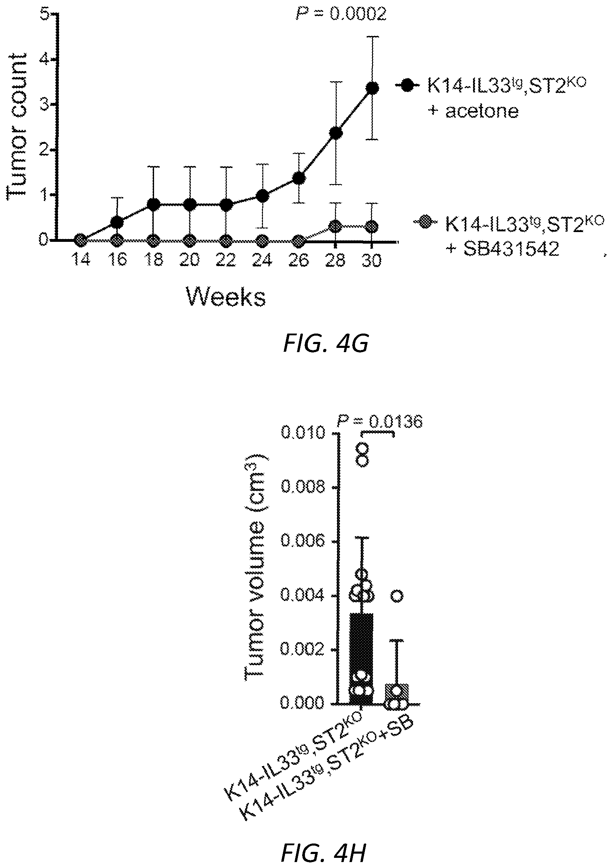

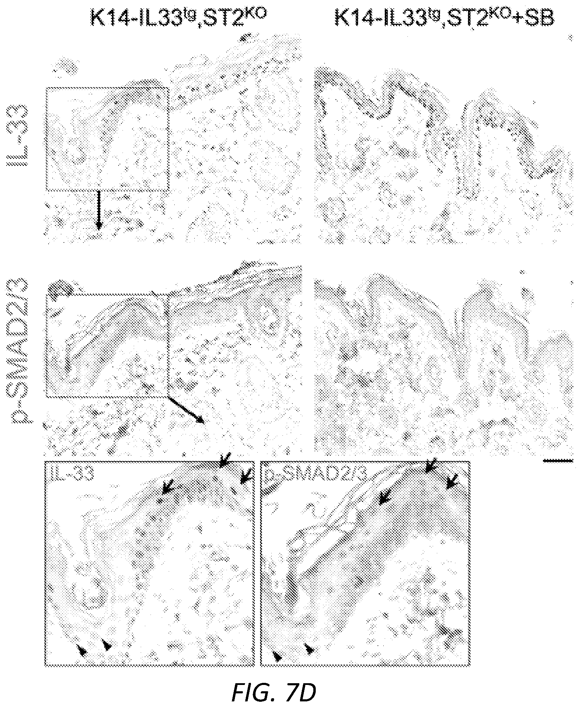

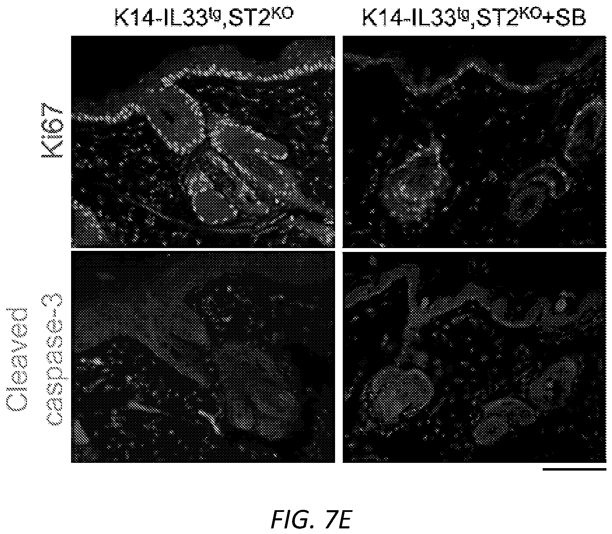

To examine the specific impact of nuclear IL-33/SMAD signaling on skin cancer, we used the standard skin carcinogenesis protocol in which mice were treated with one dose of DMBA followed by topical application of 12-O-tetradecanoylphorbol-13-acetate (TPA) three times a week for 30 weeks (Fig 7A). TPA treatment caused a significantly less IL-33 induction in the skin compared with DNFB treatment (Fig 7B). Considering the low levels of nuclear IL-33 in WT skin keratinocytes (Fig 4A), blocking the TGF-β/SMAD signaling pathway by SB431542 over the 30-week DMBA/TPA treatment had no significant impact on skin tumor onset, tumor numbers over time, or endpoint tumor volumes in WT mice (Fig 4B-D and Fig 7C). To study the impact of TGF-β/SMAD signaling on skin cancer development in the presence of increased nuclear IL-33 while excluding IL-33 cytokine function, we generated K14-IL33^{tg},ST2^{KO} mice that overexpressed IL-33 specifically in their skin keratinocytes but lacked IL-33 cytokine receptor (ST2) on all their cells (Fig 4E). K14-IL33^{tg},ST2^{KO} mice treated with SB431542 showed markedly increased tumor latency and developed significantly fewer skin tumors compared with acetone-treated mice in response to DMBA/TPA treatment (Fig 4F and G). Moreover, skin tumors that developed in K14-IL33^{tg}ST2^{KO} mice treated with DMBA/TPA and SB431542 were smaller than those developed in DMBA/TPA-treated K14-IL33^{tg},ST2^{KO} controls (Fig 4H and Fig 7C). SB431542 treatment reduced the number of p-SMAD2/3⁺ and Ki67⁺ epidermal keratinocytes without impacting apoptosis in DMBA/TPA-treated K14-IL33^{tg},ST2^{KO} skin compared with the acetone-treated controls (Fig 7D and E). Consistent with the specific requirement for TGF-β/SMAD signaling in K14-IL33^{tg}ST2^{KO} skin tumor promotion, DMBA/TPA-treated K14-IL33^{tg},ST2^{KO} skin had higher expression of both p-SMAD2/3 and p-SMAD1/5 and lower expression of SMAD6 compared with WT mice (Fig 4I and Fig 8A). These results demonstrate that IL-33 induction promotes skin cancer development via the TGF-β/SMAD signaling pathway. To determine whether IL-33/SMAD signaling was also active in human skin cancer, we examined IL-33 and p-SMAD2/3 protein levels in human skin cancer versus normal tissues. Consistent with our findings in mice, IL-33 and p-SMAD2/3 were significantly upregulated in the nuclei of human cutaneous squamous cell carcinoma (SCC) cells compared with normal skin keratinocytes (Fig 4J and Fig 8B and C).

### Example 1.5. Nuclear IL-33 promotes pancreatic cancer development in chronic pancreatitis

To extend our findings to other epithelial cancers affected by chronic inflammation, we investigated the role of nuclear IL-33 in pancreatic cancer. Chronic pancreatitis is a common risk factor for developing pancreatic cancer (Bansal & Sonnenberg, 1995; Guerra *et al,* 2007). To examine the expression and localization of IL-33 in cancer-prone chronic pancreatitis, we treated WT mice with intraperitoneal injections of caerulein in phosphate-buffered saline (PBS) or PBS alone hourly for 6 hours per day, three days per week for 4 weeks (Fig 9A) (Lin *et al,* 2014). Caerulein treatment led to the development of chronic pancreatitis and a significant upregulation of IL-33 in the WT pancreas (Fig 9B and C). IL-33 and p-SMAD2/3 were highly increased in the nuclei of pancreatic acinar cells in caerulein- compared with PBS-treated WT mice (Fig 5A). Consistent with the nuclear function of IL-33 in epithelial cells, *Il33* upregulation associated with a marked reduction in *Smad6* expression in caerulein-compared with the PBS-treated pancreas (Fig 5B). Furthermore, p-SMAD2/3 and p-SMAD1/5 were increased and SMAD6 was decreased in caerulein- compared with PBS-treated pancreas (Fig 5C and Fig 9D). These results suggest that nuclear IL-33 via the induction of TGF-β/SMAD signaling may promote the transformation of pancreatic acinar cells to PDAC in the context of chronic pancreatitis.

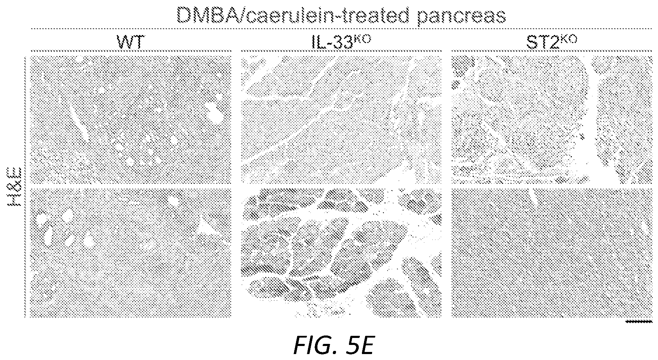

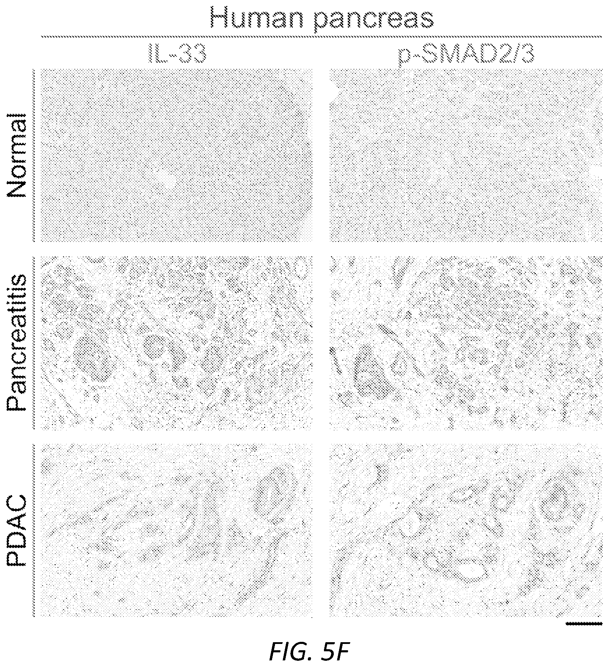

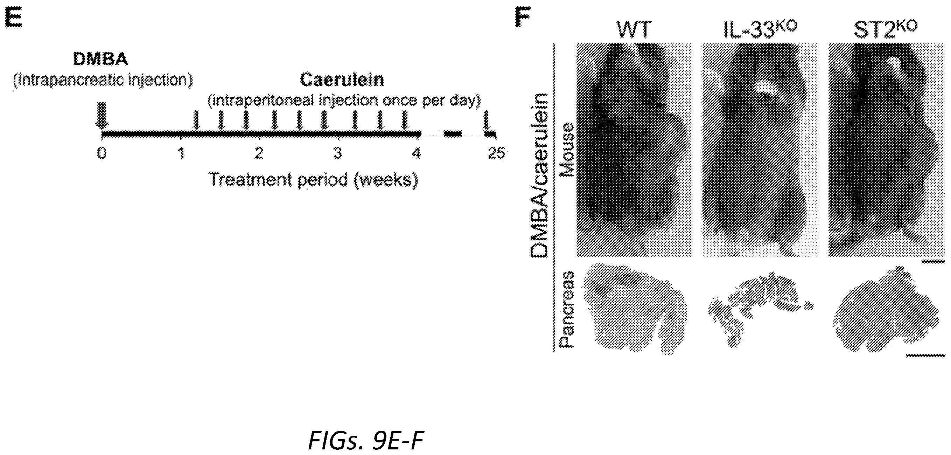

To examine the impact of nuclear IL-33 on pancreatic cancer development in chronic pancreatitis, IL-33^{KO}, ST2^{KO}, and WT mice received an intrapancreatic injection of DMBA followed by intraperitoneal injections of caerulein once a day, three-time a week for 25 weeks (Fig 9E) (Guerra *et al.,* 2007; Scarlett *et al,* 2011). DMBA/caerulein-treated WT mice developed PDAC while IL-33^{KO} mice only showed mild pancreatitis with no pancreatic cancer detectable at the completion of the 25-week DMBA/caerulein treatment (Fig 5D and E, and Fig 9F). DMBA/caerulein treatment caused severe pancreatitis in ST2^{KO} mice, which led to their early demise compared with IL-33^{KO} animals (Fig 5D). Post-mortem analysis revealed severe fibrotic tumors in the pancreas of ST2^{KO} mice (Fig 5E and Fig 9F). To determine whether IL-33/SMAD signaling is active in pancreatitis-associated PDAC in humans, we examined IL-33 and p-SMAD2/3 expression in the nuclei of the epithelial cells across 18 matched samples of the normal pancreas, pancreatitis and pancreatitis-associated PDAC. IL-33 and p-SMAD2/3 were highly expressed in the epithelial cells in pancreatitis and pancreatitis-associated PDAC samples (Fig 5F-H). Moreover, the number of IL-33⁺ epithelial cells was positively correlated with the number of p-SMAD2/3⁺ epithelial cells across the samples (Fig 5I). Collectively, these outcomes demonstrate that the nuclear IL-33/SMAD signaling axis promotes cancer development in the context of chronic inflammation in the skin and pancreas.

### Example 2. Pitavastatin and tropisetron are novel small molecule inhibitors of IL-33

The number of patients suffering from severe inflammatory diseases and cancer is on the rise. Asthma, atopic dermatitis and food allergy are common inflammatory diseases that are linked to Interleukin (IL)-33 overexpression as the driving etiology. IL-33 leads to a type 2 immune response including Th2 cells and their associated cytokines, IL-4, 5 and 13. Furthermore, **IL-33** receptor (ST2) are expressed by several immune cells, like regulatory T cells, macrophages and mast cells. Because of diverse role of IL-33 with immune cells in chronic inflammation, many bio-pharma companies are investigating of blocking antibody for IL-33. Small molecule has a lot of advantage compared to antibody, but there is no small molecule inhibitor of IL-33 available. Here, by using a luciferase screening method, we found novel FDA-approved drugs, **pitavastatin** calcium (016) and **tropisetron** (F3), which can inhibit IL-33 expression. Treatment with pitavastatin decreased Poly I:C induced- and endogenous IL-33 expression *in vitro.* Furthermore, pitavastatin treatment decreased IL-33 expression in DNFB-treated mice. So, pitavastatin and tropisetron are candidate drugs for treatment of chronic inflammation and cancer.

### Materials and Methods

The following materials and methods were used in Example 2.

### Cell lines, vector and reagents

Pam212 cells were grown at 37°C in DMEM (Thermo Fisher Scientific, Waltham, MA, catalog no.11995065) supplemented with 10% fetal bovine serum (Thermo Fisher Scientific, catalog no. 26140079), 1X penicillin-streptomycin-glutamine (Thermo Fisher Scientific, catalog no. 10378016), 1X MEM non-essential amino acids solution (Thermo Fisher Scientific, catalog no. 11140050), 1X HEPES (Thermo Fisher Scientific, catalog no. 15630080) and 0.1% 2-Mercaptoethanol (Thermo Fisher Scientific, catalog no. 21985023). PyMT cell lines (derived from a primary breast tumor of NMTV-PyMT^{tg} mouse on a C57BL/6 background) were grown at 37 °C in RPMI Medium 1640 (Thermo Fisher Scientific, catalog no. 11875093) supplemented with 10% fetal bovine serum, 1X penicillin-streptomycin-glutamine and 0.1% 2-Mercaptoethanol. IL-33 promoter-luci vector (GeneCopoeia, Rockville, MD, catalog no. MPRM3494-PG02) or Control-luci vector (deletion form of IL-33 promoter region from IL-33 promoter-luci vector) or Poly (I:C) (Invivogen, San Diego, CA, catalog no. tlrl-pic) in Pam212 cells were performed using Lipofectamine 2000 (Thermo Fisher Scientific, catalog no. 11668019) according to the manufacturer's instructions.

### Stable cell lines

After transfection of plasmids in Pam212 cells, cells were incubated with 3 µg/ml of puromycin (Invivogen, San Diego, CA, catalog no. ant-pr-1) for positive transfected cell selection. After following 2 days, medium was changed new puromycin contained medium for further selection. After following 2 days, cells were moved to new plate with puromycin contained medium. Puromycin selection was repeated for five times more.

### Small compound with Luciferase assay

1000 stable cells were seed to 384 wells (Corning, Glendale, AZ, catalog no. 3570) and each of plate was incubated 24 hours. Following day, 10 µM of FDA-approved Drug Library compounds (Selleckchem, Houston, TX, catalog no. L1300) were added to each well. After 24 hours, 5 µL of luciferase buffer from Pierce Gaussia luciferase glow assay kit (Thermo Fisher Scientific, Waltham, MA, catalog no.16160) were added each well and incubated for 5 min. Each plate was read and measured with luminescence by EnVision 2014 plate reader (Perkin Elmer, Waltham, MA, catalog no. 2014 EnVision).

### Western blot

Cell lysates were prepared in LIPA buffer (Thermo Fisher Scientific, catalog no. 89900) consisting of 1X protease inhibitor cocktail, EDTA-free (Thermo Fisher Scientific, catalog no. A32955). Mice tissues were meshed and lysed by 0.1% TWEEN-20 (Millipore Sigma, catalog no. P1379) in PBS. Mice tissues were frozen by liquid nitrogen and thaw by incubation at 37 °C for further lysis. Cell lysates were sonicated for 20 sec, and then they were centrifugated at 13,200 rpm. After checking the protein concentration of each sample, the same amount of proteins was loaded into Mini-PROTEIN TGX^{™} Gels (BIO-RAD, Hercules, CA, catalog no.456-1083 or 456-1086) with 1X Tris/Glycine/SDS buffer (BIO-RAD, catalog no.1610732). A few minutes later (according to protein size), they were transferred to Immobilon-P membrane (Millipore Sigma, catalog no. IPVH00010) with Transfer buffer (Boston Bioproducts, Ashland, MA, catalog no. BP-190). Then, they were incubated 3% bovine serum albumin (Thermo Fisher Scientific, catalog no. BP1600) or 5% Skim milk (BD biosciences, San Jose, CA, catalog no. 232100) in 1X Tris-Buffered Saline (Boston Bioproducts, catalog no. BM301X) containing 0.1% TWEEN, called TBS-T for 30 min. After the membrane was washed with TBS-T, they were subjected to immunoblot with proper antibodies for overnight at 4 °C. The following day, they were incubated with proper secondary antibodies and were developed with Pierce ECL Western blotting substrate kit (Thermo Fisher Scientific, catalog no. 32106).

### Quantitative PCR

Mouse dorsal skin samples were homogenized and lysed in RLT lysis solution (QIAGEN, catalog no. 79216)/0.1% MeOH using Mini-BeadBeater-8 (BioSpec Products, Inc., Bartlesville, OK). Total RNA was extracted using an RNeasy micro kit and quantified using NanoDrop ND-1100 (NanoDrop Technologies, Wilmington, DE). cDNA samples were synthesized from 1 µg of total RNA using Invitrogen SuperScripts III Reverse Transcriptase (Thermo Fisher Scientific, catalog no. 18080085). Expression levels of all cDNA samples were determined with LightCycler 480 system (Roche Molecular Systems Inc., Pleasanton, CA) using TaqMan Universal Master Mix II (Thermo Fisher Scientific, catalog no. 44-400-40). The TaqMan analysis was performed in a final reaction of 10 µL with 4.5 µL cDNA and 5.5 µL TaqMan master mix and corresponding primers (20 µM). All assays were conducted in triplicate and normalized to *Gapdh* expression.

### Skin chronic inflammation

Four- to six-week old male and female mice were shaved on their abdomen and sensitized to 50 µL 0.5% 1-Fluoro-2,4-dinitrobenzene (DNFB, Millipore Sigma, St. Louis, MO, catalog no. 42085) dissolved in acetone plus olive oil at 3:1 ratio (refer to as acetone). Two days after first sensitization, mice were re-sensitized to 50 µL 0.25% DNFB on their abdomen. After 5 days, mice were challenged with 100 µL 0.25% DNFB on their back skin, which was repeated three times per week for 22 days. Skin rash was monitored over the duration of the study.

### Treatment with pitavastatin on skin

Mice were treated with 0.5 mM pitavastatin in 200 µL 70% ethanol or 200 µL 70% ethanol alone to their back skin twice a week. Pitavastatin/70% ethanol treatments were given in DNFB doses.

### Oral gavage with pitavastatin

4 mg/kg of pitavastatin was dissolved by olive oil and mice were administered 100 µL of pitavastatin by using metal gavage needles once a week.

### Chronic pancreatitis

Mice were weighed and injected with 50 µg/kg caerulein (BACHEM, Torrance, CA, catalog no. 4030451) in 100 µL of PBS intraperitoneally every hour for 6 hours, three days per week for 3 weeks and mice were harvested.

### Injection with pitavastatin

1 mM of pitavastatin was dissolved in DMSO for stock, and then 100 uM was dissolved in PBS with caerulein for mice injection. Mice were injected with caerulein.

### ChIP assay

Pam212 cells that had been transfected with plasmids were fixed in 1% formaldehyde (Millipore Sigma, catalog no. F8775) for 10 min and were washed by cold PBS. Cells were lysed with buffer contained with 2.5% of glycerol, 50 mM HEPES (pH 7.5), 150 mM NaCl, 0.5 mM EDTA, 0.5% NP-40 and 0.25% Triton X-100. After centrifugation at 2,000 rpm, cells lysate were resuspended in buffer A contained with 1 mM Tris/HCl (pH 7.9), 20 mM NaCl and 0.5 mM EDTA and incubated at room temperature for 10 min. Following the second centrifugation at 2,000 rpm, cells were sonicated in sonication buffer containing 10 mM HEPES, 1 mM EDTA and 0.5% SDS for 30 min to achieve chromatin fragmentation. Following centrifugation at 13,000 rpm, proteins were immunoprecipitated with HA antibody at 4 °C overnight. Next day, the samples were incubated with 30 µL 50% slurry of ChIP-Grade Protein G agarose beads (Cell Signaling Technology, catalog no. 9007S) for 2 hours. Samples were precipitated, washed three times with wash buffer containing 20 mM Tris/HCl (pH 7.9), 10 mM NaCl, 2 mM EDTA, 0.1% SDS and Triton X-100. After the last wash step, samples were eluted with 100 µL of TE buffer contained with 100 mM Tris/HCl (pH 8), 1mM EDTA and 1% SDS for three times. De-crosslinking was performed by overnight incubation with 15 µL of 3 M NaCl at 65°C. Precipitated DNA was purified by ChIP DNA Clean & Concentrator (ZYMO research, Irvine, CA, catalog no. D5205) and subjected to quantitative PCR.

### Nuclear extraction

Pam212 cell line was transfected with poly (I:C) using Lipofectamine 2000 according to the manufacturer's instructions. After 8 hours, cells were lysed using Nuclear Extraction Kit (Novus biologicals, Littleton, CO, catalog no. NBP2-29447). Briefly, cells were washed with 1XPBS supplemented with PMSF (Novus biologicals, catalog no. KC-407). Cells were centrifuged for 5 min at 1,000 rpm. Cell pellets were resuspended in 1X cold hypotonic buffer (Novus biologicals, catalog no. KC-401) and incubated on ice for 15 min. After adding 10 µL of the 10% detergent solution (Novus biologicals, catalog no. KC-403), cell lysates were centrifuged 10 min at 14,000 rpm. The supernatants were kept as cytoplasmic fraction and the cell pellets were resuspended in 100 µL nuclear lysis buffer (Novus biologicals, catalog no. KC-402). Lysates from the pellets were sonicated for few seconds and incubated for 10 min at 4°C. Then, lysates were centrifuged at 14,000 rpm for 10 min. The supernatants were kept as nuclear fraction.

### Histology and immunohistochemistry

Tissue samples were collected and fixed in 4% paraformaldehyde (Millipore Sigma, catalog no. P6148) overnight at 4°C. Next, tissues were dehydrated in ethanol, processed and embedded in paraffin. 5 µm sections paraffin-embedded tissues were placed on slides, deparaffinized and stained with H&E or toluidine blue (for mast cell detection). For immunohistochemistry, antigen retrieval was performed in 500 µL of antigen unmasking solution (VECTOR Laboratories, Burlingame, CA, catalog no. H3300) diluted in 50 mL water using Cuisinart pressure cooker for 20 min at high pressure. Slides were washed three times for three minutes each in 1X TBS with 0.025% Triton X-100. Sections were blocked with 1% bovine serum albumin (Thermo Fisher Scientific, catalog no. BP1600) and 5% goat serum (Millipore Sigma, catalog no. G9023) for 1 hour. Slides were stained overnight at 4°C with a primary antibody diluted in TBS containing 1% BSA (Appendix Table S2). The following day, slides were washed as above and incubated in 100 µL biotinylated secondary antibody (VECTOR Laboratories, catalog no. PK-6200) for 30 min. After washing, slides were incubated with 100 µL mixture of reagent A and B from VECTASTAIN Elite ABC universal kit Peroxidase (VECTOR Laboratories, catalog no. PK-6200) for 30 min. After washing, slides were incubated with 100 µL ImmPACT DAB chromogen stating (VECTOR Laboratories, catalog no. SK-4105) for a few minutes. Finally, slides dehydrated in ethanol and xylene and mounted with a coverslip using 3 drops of mounting media.

### Immunofluorescence

For immunofluorescence staining, Pam212 cells were permeated with 1XPBS supplemented with 0.2% Triton X-100 for 5 min. Slides were washed three times for three minutes each in 1XPBS with 0.1% TWEEN20. Slides were blocked with 1% bovine serum albumin and 5% goat serum for 1 hour. The slides were stained overnight at 4°C with IRF3 antibody. The following day, slides were washed as above and incubated for two hours at room temperature with secondary antibodies conjugated to fluorochromes. Next, slides were incubated with 1:2000 DAPI (Thermo Fisher Scientific, catalog no. D3571) for five minutes at room temperature, then washed as above. Slides were mounted with Prolong Gold Antifade Reagent (Thermo Fisher Scientific, catalog no. P36930).

**Table B.**

| **Western blotting Antibodies** | **Clone** | **Company** |
|---|---|---|
| ***First antibodies*** | | |
| Anti-mouse IL-33 | Polyclonal | R&D Systems, Minneapolis, MN |
| Anti-IRF3 | D83B9 | Cell Signaling Technology, Danvers, MA |
| Anti-p-IRF3 | D6O1M | Cell Signaling Technology, Danvers, MA |
| Anti-GAPDH | D16H11 | Cell SignalingTechnology, Danvers,MA |

| ***Secondary antibodies*** | **Cat** # | **Company** |
|---|---|---|
| Peroxidase Goat Anti-Rabbit IgG | 111-035-003 | Jackson Immunoresearch, West Grove,PA |
| Peroxidase Bovine Anti-Goat IgG | 805-035-180 | Jackson Immunoresearch, West Grove,PA |

| **Immunostaining antibodies** | **Clone** | **Company** |
|---|---|---|
| Anti-IL-33 (mouse) | Nessy-1 | ENZO, Farmingdale, NY |
| Anti-IRF3 | SL-12 | Santa Cruz Biotechnology, Dalla, TX |

| ***Staining kits*** | **Cat** # | **Company** |
|---|---|---|
| Antigen unmasking solution | H3300 | Vector Laboratories, Burlingame, CA |
| VECTASTAIN Elite ABC unive rsal Kit | PK6200 | Vector Laboratories |
| ImmPACT DAB chromogen statting kit | SK-4105 | Vector Laboratories |

| ***Immunofluorescence stainingAntibodies*** | **Clone** | **Company** |
|---|---|---|
| Anti-IRF3 | D83B9 | Cell Signaling Technology, Danvers, MA |

| ***Secondary antibodies*** | **Cat** # | **Company** |
|---|---|---|
| Goat anti-Rabbit IgG, Alexa Fluor 488 conjugate | A32731 | Thermo Fisher Scientific, Waltham, MA |

| **Primer information** | | |
|---|---|---|
| ***qPCR primers:* Taqman** | **Cat** # | **Company** |
| *Il33* | Mm.PT.58. 12022572 | Integrated DNA Technologies,Coralville, Iowa |
| *Gapdh* | Mm.PT.39a.1 | Integrated DNA Technologies, Coralville, Iowa |

| **SYBR green** | | |
|---|---|---|
| *Il33 promoter forward* | 5'-GAAACTCATGCAGACTGTTGACCAA-3' (SEQ ID NO:20) | |
| *Il33 promoter reverse* | 5'-GTGCACGATTCTTAGAAAATGTT-3' (SEQ ID NO:21) | |

### Example 2.1. Small Molecule Inhibitors of IL-33

To make small molecule screening platform, we generated both IL-33 promoter-luci and control-luci over-expressed stable epithelial cell line (Pam212) by multiple puromycin selection strategy (Fig. 10A). To find molecules that can inhibit IL-33 expression, we tested 1056 FDA-approved small molecules against our reporter cell line. Finally, we narrowed down to five small molecule candidates by calculating IL-33/Control luminescence ratio (O16: pitavastatin calcium, F3: tropisetron, C22: ammonium glycyrrhizinate, H13: ticagrelor, and L5: cetrimonium bromide; Fig. 10B). To find bona fide inhibitors of IL-33, we checked RNA level of *Il33* by qPCR in cells treated with inducer of IL-33 expression, Poly (I:C). After cells were treated by Poly (I:C), RNA level of *Il33* was increased compared to carrier group. However, *Il33* RNA level was decreased by pitavastatin and tropisetron in Poly I:C-treated cells (Fig. 10C and D). Moreover, treatment with pitavastatin and tropisetron showed inhibitory effect to endogenous *Il33* RNA level in breast cancer cell line, PyMT (Fig. 10E). Finally, endogenous IL-33 protein level was decreased after treatment with pitavastatin in a dose-dependent manner (Fig. 10F). Therefore, pitavastatin is a small inhibitor molecule of IL-33.

Next, to investigate the mechanism by which pitavastatin can block IL-33 expression, we first confirmed p-IRF3 expression level because IRF3 is a transcription factor of IL-33 expression. After Pam212 cells were treated by Poly (I:C), p-IRF3 was highly induced compared to carrier group. However, pitavastatin treatment with Poly (I:C) to Pam212 cells, level of p-IRF3 was decreased (Fig. 11A). We also confirmed pitavastatin treatment significantly reduced Poly (I:C) induced p-IRF3 bindings to *Il33* promoter region compared to only Poly (I:C) treated group (Fig. 11B). After IRF3 are phosphorylated by upstream, p-IRF3 can translocate to nucleus as a transcription factor. To confirm pitavastatin effects on IRF3 localization, we tested IRF3 nuclear immunofluoresence staining. pitavastatin treatment blocked IRF3 nuclear localization compared to only Poly (I:C) treated group (Fig. 11C). Moreover, treatment of pitavastatin remains IRF3 in cytoplasm by confirming nuclear fractionation assay (Fig. 11D).

Next, we also investigated effect of pitavastatin *in vivo* chronic inflammation model. DNFB treated mice had severe chronic inflammation in back skin, but the pitavastatin treated group had mild chronic inflammation compared to only DNFB treated mice (Fig. 11E). Consistent with these results, the pitavastatin-treated group decreased IL-33 expression in both RNA and protein level (Fig. 11F). Moreover, the pitavastatin-treated group showed decreased p-IRF3 compared to only DNFB treated group (Fig. 11G and H). Next, we studied the effect of pitavastatin treatment on chronic inflammation in mice. Topical treatment with DNFB plus pitavastatin showed reduced epidermal thickness and reduced mast cell infiltration compared to DNFB plus carrier only treatment (Fig. 12A-C). Furthermore, both IL-33 and nuclear IRF3 were reduced in same epithelial region of DNFB plus pitavastatin versus DNFB plus carrier treated mice (Fig. 12D and E). Thus, pitavastatin is a small molecule IL-33 inhibitor that suppresses skin inflammation.

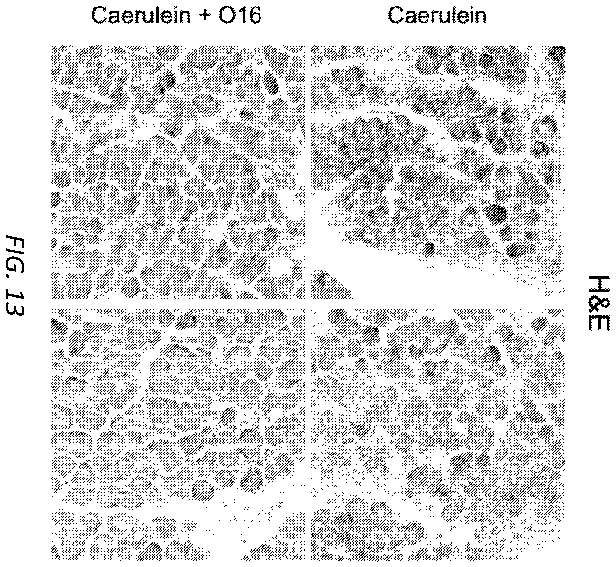

Finally, we investigated effect of pitavastatin in a pancreatitis model. Like skin inflammation result, pitavastatin treatment with caerulein showed mild pancreatitis compared to severe pancreatitis in caerulein plus carrier injected mice (Fig. 13). Therefore, pitavastatin can decrease severity of both skin and pancreas chronic inflammation. Pitavastatin can be a novel treatment for IL-33 mediated chronic inflammation, like asthma or atopic dermatitis, allergic contact dermatitis, hepatitis, or pancreatitis.

### Example 3. Role of IL-33 in Development of Chronic Hepatitis and Hepatocellular Carcinoma (HCC).

To investigate the role of HBV infection in chronic hepatitis and HCC, we developed a model of chronic HBV infection in mice. The hydrodynamic delivery of pAAV-HBV1.2 into the animal's liver led to the long-term persistence of HBsAg in the sera of the animals (Fig. 6A). Chronic HBV infection alone did not cause hepatitis or HCC in mice; however, HBV infection significantly amplified the inflammation and HCC development in response to DEN exposure (Fig. 6B). Importantly, HBV/DEN-treated liver markedly upregulated IL-33 expression in the hepatocytes at 4 months post-infection and before the onset of HCC development (Fig. 6C). Deletion of IL-33 suppressed HCC in the animals (Fig. 6, D and E). These outcomes reveal a critical role for IL-33 in the promotion of chronic hepatitis and HCC.

### Example 4. IL-33 Promotes Skin Fibrosis

Interleukin (IL)-33 is found to be upregulated in tissue fibrosis associated with human systemic sclerosis (SSc), which suggests its involvement in disease pathogenesis (Li et al, 2018; Molofsky et al., 2015). Specifically, the production of IL-33 by activated fibroblasts and myofibroblasts has been associated with the fibrotic changes seen in SSc (Koca et al., 2016; Manetti et al., 2010; Zhang et al., 2018). However, the precise role of IL-33 in tissue fibrosis remains unclear.

### Materials and Methods

The following materials and methods were used in Example 4.

### Mice

All mice used in this study were housed in pathogen-free conditions at the Massachusetts General Hospital animal facility in compliance with animal care regulations. The study was approved by Massachusetts General Hospital IACUC. C57BL/6 WT mice were from the Jackson Laboratory (Bar Harbor, ME). IL-33^{KO} mice were a gift from Dr. Marco Colonna, Washington University in St. Louis.

### BLM-induced skin fibrosis

Six- to ten-week-old female mice were anesthetized with isoflurane before being subcutaneously injected on the shaved back with sterilized PBS control or 50 µg BLM (Millipore Sigma, St. Louis, MO, catalog no. B1141000) dissolved in sterilized PBS at the same spot every other day for 27 days (Fanny et al., 2018). Skin samples from the injection site were collected for tissue analysis.

### Example 4.1

We demonstrate that in bleomycin-induced skin fibrosis (Kalekar et al., 2019), IL-33 overexpression in dermal fibroblasts co-localizes with phospho-SMAD2/3 (Fig. 15A). This suggests that nuclear function of IL-33 promotes TGF-β signaling in fibroblasts as we have shown in cancer (see Examples 1-2, above). TGF-β signaling activation is a dominant driver of fibrosis (Budi et al., 2021). Importantly, IL-33 deletion suppresses bleomycin-induced skin fibrosis (Fig. 15B).

These findings demonstrate that small molecule IL-33 inhibitors can suppress fibrosis.

### References

Ali S, Mohs A, Thomas M, Klare J, Ross R, Schmitz ML, Martin MU (2011) The dual function cytokine IL-33 interacts with the transcription factor NF-kappaB to dampen NF-kappaB-stimulated gene transcription. J Immunol 187: 1609-1616
Ameri AH, Moradi Tuchayi S, Zaalberg A, Park JH, Ngo KH, Li T, Lopez E, Colonna M, Lee RT, Mino-Kenudson M et al (2019) IL-33/regulatory T cell axis triggers the development of a tumor-promoting immune environment in chronic inflammation. Proceedings of the National Academy of Sciences of the United States of America 116: 2646-2651
Bansal P, Sonnenberg A (1995) Pancreatitis is a risk factor for pancreatic cancer. Gastroenterology 109: 247-251
Bardeesy N, Cheng KH, Berger JH, Chu GC, Pahler J, Olson P, Hezel AF, Horner J, Lauwers GY, Hanahan D et al (2006) Smad4 is dispensable for normal pancreas development yet critical in progression and tumor biology of pancreas cancer. Genes & development 20: 3130-3146
Brahmer JR, Tykodi SS, Chow LQ, Hwu WJ, Topalian SL, Hwu P, Drake CG, Camacho LH, Kauh J, Odunsi K et al (2012) Safety and activity of anti-PD-L1 antibody in patients with advanced cancer. The New England journal of medicine 366: 2455-2465
Budi EH, J. R. Schaub, M. Decaris, S. Turner, R. Derynck, TGF-beta as a driver of fibrosis: physiological roles and therapeutic opportunities. The Journal of pathology 254, 358-373 (2021)
Carriere V, Roussel L, Ortega N, Lacorre DA, Americh L, Aguilar L, Bouche G, Girard JP (2007) IL-33, the IL-1-like cytokine ligand for ST2 receptor, is a chromatin-associated nuclear factor in vivo. Proceedings of the National Academy of Sciences of the United States of America 104: 282-287
Chan BCL, Lam CWK, Tam LS, Wong CK (2019) IL33: Roles in Allergic Inflammation and Therapeutic Perspectives. Front Immunol 10: 364
Chen X, Lu K, Timko NJ, Weir DM, Zhu Z, Qin C, Mann JD, Bai Q, Xiao H, Nicholl MB et al (2018) IL-33 notably inhibits the growth of colon cancer cells. Oncol Lett 16: 769-774
Colak S, Ten Dijke P (2017) Targeting TGF-beta Signaling in Cancer. Trends Cancer 3: 56-71
Fanny et al., The IL-33 Receptor ST2 Regulates Pulmonary Inflammation and Fibrosis to Bleomycin. Frontiers in immunology 9, 1476 (2018)
Grivennikov SI, Greten FR, Karin M (2010) Immunity, inflammation, and cancer. Cell 140: 883-899
Guerra C, Schuhmacher AJ, Canamero M, Grippo PJ, Verdaguer L, Perez-Gallego L, Dubus P, Sandgren EP, Barbacid M (2007) Chronic pancreatitis is essential for induction of pancreatic ductal adenocarcinoma by K-Ras oncogenes in adult mice. Cancer cell 11: 291-302
Hatzioannou A, Banos A, Sakelaropoulos T, Fedonidis C, Vidali MS, Kohne M, Handler K, Boon L, Henriques A, Koliaraki V et al (2020) An intrinsic role of IL-33 in Treg cell-mediated tumor immunoevasion. Nature immunology 21: 75-85
He Z, Chen L, Souto FO, Canasto-Chibuque C, Bongers G, Deshpande M, Harpaz N, Ko HM, Kelley K, Furtado GC et al (2017) Epithelial-derived IL-33 promotes intestinal tumorigenesis in Apc (Min/+) mice. Sci Rep 7: 5520
Hjelmeland MD, Hjelmeland AB, Sathornsumetee S, Reese ED, Herbstreith MH, Laping NJ, Friedman HS, Bigner DD, Wang XF, Rich JN (2004) SB-431542, a small molecule transforming growth factor-beta-receptor antagonist, inhibits human glioma cell line proliferation and motility. Mol Cancer Ther 3: 737-745
Imamura T, Takase M, Nishihara A, Oeda E, Hanai J, Kawabata M, Miyazono K (1997) Smad6 inhibits signalling by the TGF-beta superfamily. Nature 389: 622-626
Jakowlew SB (2006) Transforming growth factor-beta in cancer and metastasis. Cancer Metastasis Rev 25: 435-457
James D, Nam HS, Seandel M, Nolan D, Janovitz T, Tomishima M, Studer L, Lee G, Lyden D, Benezra R et al (2010) Expansion and maintenance of human embryonic stem cell-derived endothelial cells by TGFbeta inhibition is Id1 dependent. Nat Biotechnol 28: 161-166
Johnson BA, 3rd, Yarchoan M, Lee V, Laheru DA, Jaffee EM (2017) Strategies for Increasing Pancreatic Tumor Immunogenicity. Clinical cancer research : an official journal of the American Association for Cancer Research 23: 1656-1669
Kalekar et al., Regulatory T cells in skin are uniquely poised to suppress profibrotic immune responses. Sci Immunol 4 (2019)
Koca et al., The IL-33 gene is related to increased susceptibility to systemic sclerosis. Rheumatol Int 36, 579-584 (2016)
Kumar S, Minnich MD, Young PR (1995) ST2/T1 protein functionally binds to two secreted proteins from Balb/c 3T3 and human umbilical vein endothelial cells but does not bind interleukin 1. J Biol Chem 270: 27905-27913
Li L, H. Zhu, X. Zuo, Interleukin-33 in Systemic Sclerosis: Expression and Pathogenesis. Frontiers in immunology 9, 2663 (2018)
Lin WR, Yen TH, Lim SN, Perng MD, Lin CY, Su MY, Yeh CT, Chiu CT (2014) Granulocyte colony-stimulating factor reduces fibrosis in a mouse model of chronic pancreatitis. PLoS One 9: e116229
Maione F, et al., Long-Lasting Anti-Inflammatory and Antinociceptive Effects of Acute Ammonium Glycyrrhizinate Administration: Pharmacological, Biochemical, and Docking Studies. Molecules. 2019 Jul; 24(13): 2453.
Malik A, Sharma D, Zhu Q, Karki R, Guy CS, Vogel P, Kanneganti TD (2016) IL-33 regulates the IgA-microbiota axis to restrain IL-1alpha-dependent colitis and tumorigenesis. The Journal of clinical investigation 126: 4469-4481
Manetti et al., The IL1-like cytokine IL33 and its receptor ST2 are abnormally expressed in the affected skin and visceral organs of patients with systemic sclerosis. Ann Rheum Dis 69, 598-605 (2010)
Mangone FR, Walder F, Maistro S, Pasini FS, Lehn CN, Carvalho MB, Brentani MM, Snitcovsky I, Federico MH (2010) Smad2 and Smad6 as predictors of overall survival in oral squamous cell carcinoma patients. Mol Cancer 9: 106
Martinez VG, Hidalgo L, Valencia J, Hernandez-Lopez C, Entrena A, del Amo BG, Zapata AG, Vicente A, Sacedon R, Varas A (2014) Autocrine activation of canonical BMP signaling regulates PD-L1 and PD-L2 expression in human dendritic cells. Eur J Immunol 44: 1031-1038
Massague J (2012) TGFbeta signalling in context. Nat Rev Mol Cell Biol 13: 616-630
Massague J, Blain SW, Lo RS (2000) TGFbeta signaling in growth control, cancer, and heritable disorders. Cell 103: 295-309
Massague J, Seoane J, Wotton D (2005) Smad transcription factors. Genes Dev 19: 2783-2810
Maywald RL, Doerner SK, Pastorelli L, De Salvo C, Benton SM, Dawson EP, Lanza DG, Berger NA, Markowitz SD, Lenz HJ et al (2015) IL-33 activates tumor stroma to promote intestinal polyposis. Proceedings of the National Academy of Sciences of the United States of America 112: E2487-2496
Melzer C, Hass R, von der Ohe J, Lehnert H, Ungefroren H (2017) The role of TGF-beta and its crosstalk with RAC1/RAC1b signaling in breast and pancreas carcinoma. Cell Commun Signal 15: 19
Molofsky AB, Savage AK, Locksley RM (2015) Interleukin-33 in Tissue Homeostasis, Injury, and Inflammation. Immunity 42: 1005-1019
Moral JA, Leung J, Rojas LA, Ruan J, Zhao J, Sethna Z, Ramnarain A, Gasmi B, Gururajan M, Redmond D et al (2020) ILC2s amplify PD-1 blockade by activating tissue-specific cancer immunity. Nature 579: 130-135
Mordasky Markell L, Perez-Lorenzo R, Masiuk KE, Kennett MJ, Glick AB (2010) Use of a TGFbeta type I receptor inhibitor in mouse skin carcinogenesis reveals a dual role for TGFbeta signaling in tumor promotion and progression. Carcinogenesis 31: 2127-2135
Moussion C, Ortega N, Girard JP (2008) The IL-1-like cytokine IL-33 is constitutively expressed in the nucleus of endothelial cells and epithelial cells in vivo: a novel 'alarmin'? PLoS One 3: e3331
Natarajan C, Yao SY, Sriram S (2016) TLR3 Agonist Poly-IC Induces IL-33 and Promotes Myelin Repair. PLoS One 11: e0152163
Oshikawa K, Yanagisawa K, Tominaga S, Sugiyama Y (2002) Expression and function of the ST2 gene in a murine model of allergic airway inflammation. Clin Exp Allergy 32: 1520-1526
Perera RM, Bardeesy N (2015) Pancreatic Cancer Metabolism: Breaking It Down to Build It Back Up. Cancer Discov 5: 1247-1261
Polumuri SK, Jayakar GG, Shirey KA, Roberts ZJ, Perkins DJ, Pitha PM, Vogel SN (2012) Transcriptional regulation of murine IL-33 by TLR and non-TLR agonists. J Immunol 189: 50-60
Saenz SA, Taylor BC, Artis D (2008) Welcome to the neighborhood: epithelial cell-derived cytokines license innate and adaptive immune responses at mucosal sites. Immunol Rev 226: 172-190
Scarlett CJ, Colvin EK, Pinese M, Chang DK, Morey AL, Musgrove EA, Pajic M, Apte M, Henshall SM, Sutherland RL et al (2011) Recruitment and activation of pancreatic stellate cells from the bone marrow in pancreatic cancer: a model of tumor-host interaction. PLoS One 6: e26088
Shen W, Tao GQ, Zhang Y, Cai B, Sun J, Tian ZQ (2017) TGF-beta in pancreatic cancer initiation and progression: two sides of the same coin. Cell Biosci 7: 39
Shi Y, Massague J (2003) Mechanisms of TGF-beta signaling from cell membrane to the nucleus. Cell 113: 685-700
Sy CB, Siracusa MC (2016) The Therapeutic Potential of Targeting Cytokine Alarmins to Treat Allergic Airway Inflammation. Front Physiol 7: 214
Taniguchi S, Elhance A, Van Duzer A, Kumar S, Leitenberger JJ, Oshimori N (2020) Tumor-initiating cells establish an IL-33-TGF-beta niche signaling loop to promote cancer progression. Science (New York, NY 369
Todoric J, Antonucci L, Karin M (2016) Targeting Inflammation in Cancer Prevention and Therapy. Cancer Prev Res (Phila) 9: 895-905
Torphy RJ, Zhu Y, Schulick RD (2018) Immunotherapy for pancreatic cancer: Barriers and breakthroughs. Ann Gastroenterol Surg 2: 274-281
Wang Q, Wei X, Zhu T, Zhang M, Shen R, Xing L, O'Keefe RJ, Chen D (2007) Bone morphogenetic protein 2 activates Smad6 gene transcription through bone-specific transcription factor Runx2. J Biol Chem 282: 10742-10748
Wang W, Li Y, Lv Z, Chen Y, Li Y, Huang K, Corrigan CJ, Ying S (2018) Bronchial Allergen Challenge of Patients with Atopic Asthma Triggers an Alarmin (IL-33, TSLP, and IL-25) Response in the Airways Epithelium and Submucosa. J Immunol 201: 2221-2231
Zhang Y, Alexander PB, Wang XF (2017a) TGF-beta Family Signaling in the Control of Cell Proliferation and Survival. Cold Spring Harb Perspect Biol 9
Zhang Y, Davis C, Shah S, Hughes D, Ryan JC, Altomare D, Pena MM (2017b) IL-33 promotes growth and liver metastasis of colorectal cancer in mice by remodeling the tumor microenvironment and inducing angiogenesis. Molecular carcinogenesis 56: 272-287
Zhang Y, Liu T, Meyer CA, Eeckhoute J, Johnson DS, Bernstein BE, Nusbaum C, Myers RM, Brown M, Li W et al (2008) Model-based analysis of ChIP-Seq (MACS). Genome Biol 9: R137
Zhang et al., Elevated serum levels of interleukin-1beta and interleukin-33 in patients with systemic sclerosis in Chinese population. Z Rheumatol 77, 151-159 (2018)

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

The invention will now be defined by reference to the following clauses:
1. A method of treating inflammation associated with IL-33 activation in a subject, the method comprising administering a therapeutically effective amount of an inhibitor selected from pitavastatin, tropisetron, ammonium glycyrrhizinate, ticagrelor, and cetrimonium bromide.
2. A method of treating an allergic, fibrotic, infectious, or chronic inflammatory disease in a subject, the method comprising administering a therapeutically effective amount of a pitavastatin, tropisetron, ammonium glycyrrhizinate, ticagrelor, and cetrimonium bromide.
3. The method of clause 2, wherein the chronic inflammatory condition is asthma, atopic dermatitis, allergic contact dermatitis, hepatitis, pancreatitis, or inflammatory bowel disease (IBD).
4. The method of clause 2, wherein the subject is having anaphylaxis.
5. The method of clause 2, wherein the fibrotic disorder is organ fibrosis.
6. The method of clause 5, wherein the organ fibrosis is fibrosis of skin hepatic, pancreatic, renal, bowel, or pulmonary tissues.
7. A method of treating or reducing risk of developing cancer in a subject, the method comprising administering a therapeutically effective amount of a pitavastatin, tropisetron, ammonium glycyrrhizinate, ticagrelor, and cetrimonium bromide.
8. The method of clause 7, wherein the subject has a fibrotic or chronic inflammatory disease.
9. The method of clause 8, wherein the chronic inflammatory condition is asthma, atopic dermatitis, allergic contact dermatitis, hepatitis, pancreatitis, or inflammatory bowel disease (IBD), or the fibrotic disorder is organ fibrosis.
10.The method of clause 9, wherein the organ fibrosis is fibrosis of skin, hepatic, pancreatic, renal, bowel, or pulmonary tissues.
11.The method of any of clauses 1-10, wherein the inhibitor is pitavastatin, and the subject does not have hyperlipidemia or mixed dyslipidemia.
12.The method of any of clauses 1-10, wherein the inhibitor is tropisetron, and the subject does not have nausea and vomiting, or does not have fibromyalgia.
13. The method of any of clauses 1-10, wherein the inhibitor is ticagrelor, and the subject does not have acute coronary syndrome or coronary artery disease (CAD).
14. A pharmaceutical composition formulated for topical application comprising pitavastatin, tropisetron, ammonium glycyrrhizinate, and/or ticagrelor.
15. The pharmaceutical composition of clause 14, which is an ointment, salve, gel, balm, lotion, milk, or cream.
16. The pharmaceutical composition of clause 14 or 15, for use in treating skin inflammation, skin fibrosis, or skin cancer in a subject.
17. A pharmaceutical composition formulated for inhlataional administration application comprising pitavastatin, tropisetron, ammonium glycyrrhizinate, ticagrelor, and/or cetrimonium bromide.
18. The pharmaceutical composition of clause 17, which is a mist, a fine powder, or an aerosol spray.
19. The pharmaceutical composition of clause 18, which is packaged in a pressured container or dispenser, optionally an inhaler, insufflator, or nebulizer.
20. The pharmaceutical composition of clauses 17 to 19, for use in treating pulmonary inflammation, pulmonary fibrosis, or lung cancer in a subject.
21. An inhibitor selected from pitavastatin, tropisetron, ammonium glycyrrhizinate, ticagrelor, and cetrimonium bromide, for use in a method of treating inflammation associated with IL-33 activation in a subject.
22. An inhibitor selected from pitavastatin, tropisetron, ammonium glycyrrhizinate, ticagrelor, and cetrimonium bromide, for use in a method of treating an allergic, fibrotic, infectious, or chronic inflammatory disease in a subject.
23. The inhibitor for the use of clause 22, wherein the chronic inflammatory condition is asthma, atopic dermatitis, allergic contact dermatitis, hepatitis, pancreatitis, or inflammatory bowel disease (IBD).
24. The inhibitor for the use of clause 22, wherein the subject is having anaphylaxis.
25. The inhibitor for the use of clause 22, wherein the fibrotic disorder is organ fibrosis.
26. The inhibitor for the use of clause 25, wherein the organ fibrosis is fibrosis of skin, hepatic, pancreatic, renal, bowel, or pulmonary tissues.
27. An inhibitor selected from pitavastatin, tropisetron, ammonium glycyrrhizinate, ticagrelor, and cetrimonium bromide, for use in a method of treating or reducing risk of developing cancer in a subject.
28. The inhibitor for the use of clause 27, wherein the subject has a fibrotic or chronic inflammatory disease.
29. The inhibitor for the use of clause 28, wherein the chronic inflammatory condition is asthma, atopic dermatitis, allergic contact dermatitis, hepatitis, pancreatitis, or inflammatory bowel disease (IBD), or the fibrotic disorder is organ fibrosis.
30. The inhibitor for the use of clause 29, wherein the organ fibrosis is fibrosis of skin, hepatic, pancreatic, renal, bowel, or pulmonary tissues.
31. The inhibitor for the use of any of clauses 21-30, wherein the inhibitor is pitavastatin, and the subject does not have hyperlipidemia or mixed dyslipidemia.
32. The inhibitor for the use of any of clauses 21-30, wherein the inhibitor is tropisetron, and the subject does not have nausea and vomiting, or does not have fibromyalgia.
33. The inhibitor for the use of any of clauses 21-30, wherein the inhibitor is ticagrelor, and the subject does not have acute coronary syndrome or coronary artery disease (CAD).

## Claims

1. An compound selected from pitavastatin, tropisetron, ammonium glycyrrhizinate, ticagrelor, and cetrimonium bromide, for use in treating cancer or reducing risk of developing cancer in a subject.

2. The compound for the use of claim 1, wherein the cancer is selected from breast cancer, prostate cancer, pancreatic cancer, brain cancer, hepatic cancer, lung cancer, renal cancer, skin cancer, ovarian cancer, and colon cancer.

3. The compound for the use of claim 1 or 2, wherein the cancer is selected from renal carcinoma and melanoma.

4. The compound for the use of any one of claims 1-3, wherein the subject has a fibrotic disorder or chronic inflammatory disease.

5. The compound for the use of claim 4, wherein the chronic inflammatory disease is selected from asthma, atopic dermatitis, allergic contact dermatitis, hepatitis, pancreatitis, and inflammatory bowel disease (IBD).

6. The compound for the use of claim 4, wherein the fibrotic disorder is organ fibrosis.

7. The compound for the use of claim 6, wherein the fibrotic disease is fibrosis of skin (e.g., scleroderma or Dupuytren's disease), hepatic tissues, pancreatic tissues, renal tissues, bowel tissues (e.g., IBD-associated fibrosis), or pulmonary tissues.

8. The compound for the use of any one of claims 1-7, wherein the compound is pitavastatin.

9. The compound for the use of any one of claims 1-8, wherein the compound is pitavastatin, and the subject does not have hyperlipidemia or mixed dyslipidemia.

10. The compound for the use of any one of claims 1-7, wherein the compound is tropisetron, and optionally wherein the subject does not have nausea and vomiting, or does not have fibromyalgia.

11. The compound for the use of any one of claims 1-7, wherein the compound is ticagrelor, and optionally wherein the subject does not have acute coronary syndrome or coronary artery disease (CAD).

12. The compound of any one of claims 1-11, wherein the compound is administered together with an immunotherapy agent (e.g., a checkpoint inhibitor), a chemotherapy agent, radiotherapy, and/or resection.

13. The compound of any one of claims 1-12, wherein the compound is administered with an anti-inflammatory agent, such as a corticosteroid or non-steroidal anti-inflammatory drug (NSAID).

14. The compound of any one of claims 1-11, wherein reducing risk of developing cancer comprises reducing risk of developing cancer in a subject who has a chronic inflammatory disease.

15. The compound of any one of claims 14, wherein the chronic inflammatory disease is the chronic inflammatory disease of skin or pancreas.
